(19) Europäisches Patentamt
European Patent Office
Office européen des brevets

(11) **EP 3 174 530 B1**

(12) **EUROPÄISCHE PATENTSCHRIFT**

(45) Veröffentlichungstag und Bekanntmachung des Hinweises auf die Patenterteilung:
**29.08.2018 Patentblatt 2018/35**

(21) Anmeldenummer: **15733632.2**

(22) Anmeldetag: **03.07.2015**

(51) Int Cl.:
***A61K 9/20*** *(2006.01)*

(86) Internationale Anmeldenummer:
**PCT/EP2015/001355**

(87) Internationale Veröffentlichungsnummer:
**WO 2016/015812 (04.02.2016 Gazette 2016/05)**

(54) **DIREKT VERPRESSBARE POLYVINYLALKOHOLE**

DIRECTLY COMPRESSIBLE POLYVINYL ALCOHOLS

POLYALCOOLS DE VINYLE APTES À LA COMPRESSION DIRECTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2014 EP 14002666**

(43) Veröffentlichungstag der Anmeldung:
**07.06.2017 Patentblatt 2017/23**

(73) Patentinhaber: **Merck Patent GmbH**
**64293 Darmstadt (DE)**

(72) Erfinder:
• **OGNIBENE, Roberto**
**64297 Darmstadt (DE)**
• **BAUER, Finn**
**64625 Bensheim (DE)**
• **WEDEL, Thorsten**
**64589 Stockstadt/Rhein (DE)**
• **MODDELMOG, Guenter**
**64354 Reinheim (DE)**

(56) Entgegenhaltungen:
**WO-A1-88/07366      WO-A1-97/17947**

EP 3 174 530 B1

**Beschreibung**

Die vorliegende Erfindung betrifft direkt verpressbare Co-Mischungen zur Tablettenherstellung mit verzögerter Wirkstofffreisetzung, die Polyvinylalkohole (PVAs) und mikrokristalline Cellulosen (MCCs) enthalten. Gegenstand der Erfindung ist auch ein Verfahren zur Herstellung entsprechender direkt verpressbarer Co-Mischungen.

Stand der Technik

Polyvinylalkohol (PVA) ist ein synthetisches, flexibles Polymer welches durch alkalische Hydrolyse von Polyvinylacetat gewonnen wird. Das Polyvinylacetat wiederum erhält man durch eine radikalische Polymerisation aus Vinylacetat. Durch unterschiedliche Kettenlängen und unterschiedliche Hydrolysegrade der Polyvinylacetate kann man Polyvinylalkohole (PVAs) verschiedenster physikalischer Eigenschaften erhalten. Die PVAs werden insbesondere als Filmbildner, Haftgele und als Viskositätsmodulatoren in einer Vielzahl von Anwendungsgebieten z.B. Lacke, Papiere, Textilien, Kosmetika, etc. eingesetzt.

Von besonderem Interesse für die Pharmazeutische Industrie ist die Verwendung der PVAs in pharmazeutischen Zubereitungen wie z.B. in Ophthalmika, als Filmbildner für überzogene Tabletten, als Bindemittel in Granulaten oder als Beschichtungskomponente in Pflastern sowie auch in Drug Delivery Systemen. Ganz besonders interessant ist die Verwendung verschiedener PVA-Typen in der Formulierung von festen oralen pharmazeutischen Darreichungsformen mit einer verlängerten Wirkstofffreisetzung z.B. in sogenannten "Retardtabletten". Eine verzögerte Wirkstofffreigabe wird in solchen Polymer-haltigen pharmazeutischen Formulierungen dadurch erzielt, dass die Tabletten sich in Gegenwart von Flüssigkeit, wie im Mund oder Magen-Darmtrakt, nicht direkt auflösen sondern quellen und der Wirkstoff durch Diffusion erst nach und nach freigesetzt wird.

Durch solche galenisch modifizierten Tabletten ist es möglich den Wirkstoff aus der Darreichungsform in einer kontrollierten Art und Weise über eine längere Zeit im Körper freizusetzen, um dadurch einen therapeutisch wirksamen Blutspiegel des Medikaments über eine längeren Zeitraum (mehrere Stunden) zu halten. Die beiden wesentlichen Vorteile solcher retardierten Formulierungen sind - im Gegensatz zu Tabletten mit einer sofortigen Wirkstofffreisetzung nach Einnahme - zum einen die Vermeidung von unerwünschten ggf. auch toxischen Blut/Plasmaspiegeln des APIs als auch eine Reduzierung der Einnahmefrequenz der Tabletten (z.B. statt 3mal/täglich nur noch 1 mal/täglich) und somit einer Verbesserung der sog. "Patienten-Compliance" verbunden mit einem verbesserten therapeutischen Ergebnis der medikamentösen Behandlung.

Bekannte Polyvinylalkohole, die für die Verwendung in pharmazeutischen Formulierungen nach den verschiedenen Pharmakopoen (Pharmacopoea Europaea, Ph. Eur.; United States Pharmakopeia (USP), und der Japanischen Pharmakopoe (JP bzw. JPE)) spezifiziert sind, sind jedoch nicht oder nur unter besonderen Bedingungen direkt durch Druckeinwirkung tablettierbar. Ein besonderes Problem besteht in diesem Zusammenhang also darin, in einfacher Weise Tabletten herzustellen, die vorwiegend aus entsprechenden PVAs als Wirkstoffträger bestehen, worin der Wirkstoff homogen verteilt ist. Eine direkte Tablettierbarkeit von PVA-haltigen Formulierungen ist üblicherweise in Gegenwart von höheren Anteilen weiterer Bindemittel, wie Laktose, und von Schmiermitteln und ggfs. weiteren Additiven zu erzielen. Häufig werden Formulierungen, in denen PVAs als Wirkstoffträger eingesetzt werden, in Gegenwart von wässrigen oder alkoholischen Lösungen hergestellt. Beispielsweise ist es bekannt, entsprechende Tabletten mit verlängerter Wirkstofffreisetzung herzustellen, indem der Wirkstoff und PVA in Gegenwart von weiteren Zusätzen nach einer Nassgranulation komprimiert werden. Letzteres ist mit dem Nachteil verbunden, dass die erforderlichen Lösungsmittel unter Energieeinsatz wieder entfernt werden müssen.

Aufgabe

Wie aus dem oben Beschriebenen hervorgeht, werden um die gewünschten Retardierungseffekte zu erzielen häufig quellende Polymere als Matrix eingesetzt aus denen nach Befeuchtung z.B. im Magen und Darm der Wirkstoff zeitkontrolliert über Diffusions- und Erosionsvorgänge freigesetzt und zur Resorption zur Verfügung gestellt wird. Bekannte Beispiele für solche Polymere sind insbesondere modifizierte Cellulosen wie die Hydroxypropylmethylcellulosen (HPMCs). Bekannt für solche Retardierungseffekte sind insbesondere aber auch die Polyvinylalkohole (PVAs). PVAs kommen dann zum Einsatz, wenn z.B. Unverträglichkeitsreaktionen zwischen Wirkstoff und HPMC bestehen oder wenn die eingesetzten HPMC-Typen ein unbefriedigendes Freisetzungsprofil des Wirkstoffs zeigen. Zur schnellen Tablettenentwicklung mit Retardierungseffekt benötigt der Galeniker ein quellendes Polymer, welches direkt verpressbar ist und dennoch den Wirkstoff zeitkontrolliert freisetzt. Pulverförmige PVAs sind per se jedoch nicht direkt verpressbar - sie liefern Tabletten ungenügender Härten, die sich in der pharmazeutischen Praxis nicht handhaben lassen, da sie beispielsweise eine unerwünschte Bruchneigung oder einen zu hohen Abrieb aufweisen.

Es ist daher Aufgabe der vorliegenden Erfindung, direkt verpressbare Retardierungsmatrices zur Verfügung zu stellen, die zeitaufwendige Granulierungsverfahren überflüssig machen; d. h. Schritte die beispielsweise aus dem Befeuchten mit Granulierflüssigkeiten, mechanischem Vermischungen in Mischsystemen oder Wirbelschichtanlagen, sowie Nachtrocknungsverfahren zur Entfernung der Granulierflüssigkeiten und Siebungen bestehen, so dass Zeit und Energie eingespart werden können, aber auch teure und zeitaufwändige Investitionen in spezielle Granulieranlagen. Aufgabe der vorliegenden Erfindung ist es auch, solche vorteilhaften direkt verpressbaren Retardierungsmatrices auf Basis von PVAs zur Verfügung zu stellen. Aufgabe der vorliegenden Erfindung ist es außerdem, ein Verfahren zur Verfügung zu stellen, durch das PVAs, bzw. handelsübliche PVA-Qualitäten in einen direkt verpressbaren Zustand überführt werden können.

Kurze Beschreibung der Erfindung

Durch die vorliegende Erfindung wird dem Galeniker eine direkt verpressbare Zusammensetzung mit verlängerter Wirkstofffreisetzung, enthaltend eine Co-Mischung aus Polyvinylalkoholen (PVAs) und mikrokristallinen Cellulosen (MCCs) zur Verfügung gestellt. Vorzugsweise sind solche Mischungen Gegenstand der vorliegenden Erfindung, worin die eingesetzten Polyvinylalkohole (PVAs) und mikrokristallinen Cellulosen (MCCs) die Anforderungen der Pharmakopöen (Ph. Eur., USP und JPE erfüllen. Erfindungsgemäß können entsprechende direkt verpressbare Zusammensetzungen Polyvinylalkohole (PVAs) der Typen 18-88, 26-88 und 40-88 und alle dazwischen liegenden Qualitäten, einschließlich des Typs 28-99 gemäß JPE und Ph. Eur. enthalten. Die Lösung der Aufgabe der vorliegenden Erfindung erfolgt insbesondere durch direkt verpressbare Zusammensetzungen, enthaltend Polyvinylalkohole (PVAs), welche der Ph. Eur. entsprechen und welche durch Polymerisation von Vinylacetat und durch anschließende teilweise oder nahezu vollständige Hydrolyse des Polyvinylacetats erhalten worden sind. Besonders gut geeignet sind Zusammensetzungen, welche Polyvinylalkohole (PVAs) enthalten, die durch 85% - 89%ige Hydrolyse erhalten worden sind. In besonderer Weise sind entsprechende Zusammensetzungen geeignet, welche Polyvinylalkohole (PVAs) enthalten, bei denen es sich um wasserquellbare Harze handelt, die nach USP durch die Formel

$$(C_2H_4O)_n$$

charakterisiert sind, worin

n eine ganze Zahl im Bereich von 500 und bis 5 000 bedeutet, und welche eine durchschnittliche relative Molekülmasse im Bereich zwischen 20 000 und 150 000 g/mol besitzen, die nach Ph. Eur. eine Viskosität im Bereich von 3 - 70 mPa.s, (gemessen in einer 4%igen Lösung bei 20 °C) besitzen und welche eine Esterzahl von nicht größer als 280 mg KOH/g (Hydrolysegrad >72.2 mol%) aufweisen.

In erfindungsgemäßen direkt verpressbaren Zusammensetzungen mit verbesserten Eigenschaften liegen die beschriebenen PVAs und MCCs in einer Co-Mischung im Verhältnis in einem Bereich 2 : 1 bis 1 : 2 bezogen auf das Gewicht, bevorzugt in einem Verhältnis in einem Bereich von 2 : 1 bis 1:1 vor. Nach intensiver Vermischung weisen die hier gefundenen Co-Mischungen aus PVA mit MCCs Schüttdichten im Bereich von 0,40 - 0,48 g/ml bei Stampfdichten im Bereich von 0,55-0,63 g/ml auf.

Darüber hinaus ist auch eine wirkstoffhaltige Tablette mit verlängerter Wirkstofffreisetzung von mehreren Stunden Gegenstand der vorliegenden Erfindung, und zwar eine Tablette enthaltend eine Co-Mischung aus Polyvinylalkoholen (PVAs)und mikrokristallinen Cellulosen (MCCs) wie oben charakterisiert. Überraschend wurde gefunden, dass entsprechende wirkstoffhaltige Tabletten verzögerte Wirkstofffreisetzungen von mindestens 2 Stunden, bevorzugt über mindestens 6 Stunden, besonders bevorzugte von mindestens 8 Stunden, insbesondere bevorzugt von mindestens 10 Stunden, und ganz besonders bevorzugt von mindestens 12 Stunden aufweisen, abhängig vom eingesetzten Wirkstoff und vom Mischungsverhältnis der Polyvinylalkohole und der mikrokristallinen Cellulosen.

Insbesondere wurde gefunden, dass wirkstoffhaltige Tabletten, die eine entsprechende direkt verpressbare Zusammensetzung in Form einer Co-Mischung in einer Menge von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette enthalten, die gewünschte, verlängerte Wirkstofffreisetzung aufweisen. Vorteilhafterweise können mit solchen Zusammensetzungen bereits bei Einsatz von niedrigen Presskräften Tabletten mit besonders hohen Tablettenhärten erhalten werden die im Herstellungsprozess überraschend geringe Ausstoßkräfte erfordern. Bereits bei Einsatz einer Presskraft von 19,5 kN können Tabletten mit einer Tablettenhärte von 295,7 N erhalten werden, die nur eine Ausstoßkraft von etwa 66,7 N erfordern. Darüber hinaus weisen diese Tabletten nur geringe Friabilitäten von weniger als 1 Gew.-%, bevorzugt weniger als 0,5 Gew.-% insbesondere von weniger als 0,1 Gew.-% auf, wie durch geeignete Versuche gezeigt werden kann.

Besonders gut lassen sich unter Verwendung der beschriebenen Co-Mischungen Tabletten mit verzögerter Wirkstofffreisetzung, welche Wirkstoffe der BCS Klasse I enthalten entweder allein oder in Kombination mit anderen Wirkstoffen durch Verpressen herstellen.

Sofern eine klinische Notwendigkeit besteht können aber auch Wirkstoffe anderer BCS Klassen mit dem erfindungsgemäßen Verfahren zu direkt verpressbaren Darreichungsfromen mit einer retardierten Wirkstofffreisetzung verarbeitet werden.

Die Aufgabe der vorliegenden Erfindung wird weiterhin gelöst durch ein Verfahren zur Herstellung von direkt verpressbaren Zusammensetzungen mit verlängerter Wirkstofffreisetzung, welche eine Co-Mischung aus mikrokristallinen Cellulosen (MCCs) und Polyvinylalkoholen (PVAs) enthalten, worin Polyvinylalkohol zu einem feinkörnigen Pulver gemahlen und durch ein 800 $\mu$m Sieb gesiebt wird, und mit mikrokristalliner Cellulose (MCCs) mit mittlerer Partikelgröße $d_{v50}$ im Bereich von 60 bis 250 $\mu$m, und einer Schüttdichte im Bereich von 0,22 bis 0,38 g/cm$^3$ intensiv vermischt wird.

Detaillierte Beschreibung der Erfindung

Häufig hängt die ausreichende Wirksamkeit von Arzneimitteln von einer gleichmäßigen Dosierung ab, und erfordert eine mehrmalige Verabreichung am Tag, so dass klinisch ausreichende Wirkspiegel im Blut über längere Zeit erhalten bzw. unerwünschte Nebenwirkungen vermieden werden können. Diese Mehrfachgabe über den Tag ist jedoch bezüglich der Patientencompliance nicht erstrebenswert. Daher ist es für die Verabreichung bestimmter Wirkstoffe wünschenswert Tablettenformulierungen zur Verfügung stellen zu können, durch die eine Wirkstofffreisetzung langsam über Stunden verläuft, so dass sich bei regelmäßiger Einnahme ein weitgehend konstanter wirksamer Blutspiegel über den Tag einstellt, aber nur eine einmalige Einnahme pro Tag erforderlich ist.

Abhängig von den einzusetzenden Wirkstoffen sind die Anforderungen an die jeweilige Zusammensetzung unterschiedlich. Je nach ihren chemischen und physikalischen Eigenschaften sind andere Wirkstoffträger und Tablettierhilfsmittel zu verwenden, da nicht jeder Wirkstoff mit jedem Tablettierhilfstoff verträglich ist, bzw. aufgrund der chemischen und physikalischen Eigenschaften miteinander verarbeitet werden können.

Die Bioverfügbarkeit von Wirkstoffen kann nach einem Biopharmazeutische Klassifizierungssystem (BCS) unterteilt werden, das Mitte der 1990er Jahre in den USA durch Gordon Amidon entwickelt worden ist und inzwischen Bestandteil sowohl einer US-FDA (Food and Drug Administration) Richtlinie als auch einer Leitlinie der europäischen Arzneimittelagentur zur Beurteilung der Bioäquivalenz von Arzneimitteln ist.

Beispielsweise sind Wirkstoffe der BCS Klasse I gut lösliche Wirkstoffe mit einem hohen Permeationsvermögen. Ihre Resorption wird nur durch die Geschwindigkeit der Magen- und Darmentleerung kontrolliert. Bei Wirkstoffen, die zu dieser Klasse gehören, deren Wirksamkeit aber über den gesamten Tag erwünscht ist, ist man bestrebt, Formulierungen zu entwickeln, durch die eine verzögerte, gleichmäßige Wirkstoffabgabe ermöglicht wird.

Das biopharmazeutische Klassifizierungssystem (kurz BCS, engl.: Biopharmaceutics Classification System) beschreibt Zusammenhänge, die bei der oralen Applikation von Arzneistoffen eine wichtige Rolle spielen. Es basiert auf der Arbeit von G. Amidon und Kollegen aus dem Jahre 1995. Die Autoren beschreiben in dieser Arbeit, dass die orale Bioverfügbarkeit von Arzneistoffen hauptsächlich von deren Löslichkeit, der Auflösungsgeschwindigkeit sowie der Permeabilität durch biologische Membranen beeinflusst wird (Amidon GL, Lennernas H, Shah VP, Crison JR. A theoretical basis for a biopharmaceutic drug classification: the correlation of in vitro drug product dissolution and in vivo bioavailability.Pharm Res. 1995;12:413.)

Bei Wirkstoffen der BCS Klasse 1 ist sowohl die Löslichkeit als auch die Permeabilität hoch.

Dieses bedeutet, wenn sowohl die Löslichkeit als auch die Permeabilität des Arzneistoffs hoch sind, kann angenommen werden, dass die Absorptionsrate hauptsächlich durch die Geschwindigkeit der Magen- und Darmentleerung bestimmt wird.

Seit August 2000 findet das BCS-System Anwendung beim Zulassungsverfahren für Fertigarzneimittel der amerikanischen Zulassungsbehörde für Arzneimittel FDA (Food and Drug Administration). Unter bestimmten Voraussetzungen kann bei der Beantragung der Zulassung für Fertigarzneimittel (FAM) auf Bioverfügbarkeits- und Bioäquivalenzstudien verzichtet werden, wenn durch Anwendung des BCS-Systems nachgewiesen wird, dass das neue Fertigarzneimittel und ein bereits zugelassenes FAM desselben Arzneistoffes bioäquivalent sein müssen. Dann kann eine Befreiung (engl.: waiver) von der Verpflichtung beantragt werden, diese kostenintensiven und in diesem Fall unnötigen Studien zur Bioverfügbarkeit durchzuführen. Dazu muss der Arzneistoff in der jeweiligen Arzneiform bestimmte Anforderungen bezüglich der Hauptparameter Löslichkeit, Permeabilität und Lösungsgeschwindigkeit erfüllen.

Löslichkeit:

Die höchste Dosis des Arzneistoffs muss sich in maximal 250 mL eines wässrigen Auflösungmedium in einem pH-Wert Bereich zwischen pH 1 und pH 7,5 vollständig lösen.

Permeabilität:

Ein Arzneistoff besitzt dann eine hohe Permeabilität, wenn mindestens 90% einer verabreichten Dosis vom Körper aufgenommen werden. Dies muss durch geeignete Daten nachgewiesen werden (z.B. Massenbilanz-Studien).

Lösungsgeschwindigkeit:

Die Arzneiform muss eine schnelle Freisetzung des Arzneistoffs gewährleisten. Dies muss durch geeignete in-vitro Freisetzungstests (entweder Drehkörbchen- oder Rührblattmethode) nachgewiesen werden. Mindestens 85% der entsprechenden Dosis müssen innerhalb von 30 Minuten in drei verschiedenen Freisetzungsmedien (0,1 N HCL, Puffer pH 4,5 und Puffer pH 6,8) freigesetzt werden.

Eine Lösung des Problems , das darin besteht einen gut löslichen Wirkstoff gleichmäßig über Stunden zur Verfügung zu stellen, erscheint hier durch die Verwendung von polymeren Wirkstoffträgern möglich, wobei letztere in Gegenwart von physiologischen Flüssigkeiten, wie Speichel, oder Magen- und Darmsaft, langsam ein Gel bilden und den Wirkstoff langsam und kontrolliert durch Diffusion aus der Tablettenmatrix abgeben.

Hier bieten sich Polyvinylalkohole (PVAs) an, die als synthetische Polymere wasserquellbare Harze sind und ausgezeichnete filmbildende und emulgierende Eigenschaften besitzen und in wässrigen Lösungen ein Gel bilden. PVAs sind nach USP durch die Formel

$$(C_2H_4O)_n$$

charakterisiert ist, worin

n eine ganze Zahl im Bereich von 500 und bis 5 000 bedeutet. Je nach der Molekülgröße dieser Polymere und ihrer chemischen Zusammensetzung variieren ihre Eigenschaften sehr, insbesondere hinsichtlich der Wasserlöslichkeit, aber auch in Bezug auf die Tablettierbarkeit.

PVAs werden aus Polyvinylacetat hergestellt, wobei die funktionellen Acetat-Gruppen teilweise oder vollständig hydrolysiert werden, um funktionelle Alkoholgruppen zu erhalten. In dem Maße in dem der Grad der Hydrolyse zunimmt, nimmt die Löslichkeit des Polymers in wässrigen Medien zu, aber auch die Kristallinität und die Schmelztemperatur des Polymers nimmt zu. Darüber hinaus variiert die Glasübergangstemperatur, abhängig vom Grad der Hydrolyse.

Beispielsweise hat ein 38% hydrolysiertes Material keinen Schmelzpunkt, aber eine Glasübergangstemperatur von etwa 48 °C, wohingegen ein zu 75% hydrolysiertes Material eine Schmelztemperatur von etwa 178 °C, ein zu 88% hydrolysiertes Material einen Schmelzpunkt von ca. 196 °C und ein zu 99% hydrolysiertes Material einen Schmelzpunkt von etwa 220 °C aufweist, wobei aber das Polymer dazu neigt, sich schnell bei einer Temperatur oberhalb von 200 °C zu zersetzen.

Zur Herstellung der erfindungsgemäßen Zusammensetzungen können Polyvinylalkohole (PVAs) der Typen 18-88, 26-88 und 40-88 und alle dazwischen liegenden Qualitäten, einschließlich des Typs 28-99 gemäß JPE oder Ph. Eur. verwendet werden.

Obwohl Polyvinylalkohole in Wasser löslich sind, sind sie in fast allen organischen Lösungsmitteln nahezu unlöslich, mit Ausnahme von wenigen Lösungsmitteln, wie beispielsweise in Ethanol mit einer geringfügigen Löslichkeit. Diese Eigenschaften der Polymere machen es sehr schwierig, Tablettenformulierungen herzustellen, in denen PVAs zu einem hohen Anteil enthalten sind und die direkt tablettierbar sind.

Für die Verwendung in pharmazeutischen Formulierungen sind nach den verschiedenen Pharmakopoen Polyvinylalkohole unterschiedlicher Hydrolysegrade spezifiziert.

Die Europäische Pharmacopoeia schreibt vor, dass ein zulässiges Polyvinylalkohol zur Verwendung in pharmazeutischen Dosierungen eine Esterzahl von nicht mehr als 280 und eine mittlere relative Molekülmasse zwischen 20.000 und 150.000 haben muss. Der Prozentsatz der Hydrolyse (H) kann aus der folgenden Gleichung berechnet werden:

$$H = ((100-(0.1535)(EV))/(100-(0.0749)(EV)))x100$$

wobei EV der Ester-Zahl des Polymers entspricht. Unter der Esterzahl ist die Angabe der Menge Kaliumhydroxid in mg gemeint, die zur Verseifung der Ester in 1 g Probe erforderlich ist. Die Esterzahl berechnet sich aus der Differenz von Verseifungs- und Säurezahl.

Somit sind nach der Monographie des Europäischen Arzneibuches nur PVA-Polymere mit einer prozentualen Hydrolyse von mehr als 72,2% einsetzbar.

Nach der United States Pharmacopeia müssen zur Verwendung in pharmazeutischen Darreichungsformen geeignete Polyvinylalkohole einen prozentualen Hydrolysegrad zwischen 85 und 89% aufweisen und einen Polymerisationsgrad von 500 bis 5000 haben. Der Polymerisationsgrad (DM) wird durch die Gleichung berechnet:

$$DM = (Molar\ Mass)/((86)-(0.42(the\ degree\ of\ hydrolysis)))$$

Ein nach der Europäischen Arzneibuch-Monographie in pharmazeutischen Formulierungen einsetzbares PVA ist ein PVA mit einem Hydrolysegrad zwischen 72,2% und 90% wodurch sowohl PVAs nach der Ph.Eur. (Hydrolyse von mehr als 72,2%, aber weniger als 90% als auch solche gemäß der USP (Hydrolysegrad zwischen 85 - 89 %) umfasst sind. Diese PVA-Qualitäten haben ein Molekulargewicht im Bereich von 14.000 g / mol bis 250.000 g / mol.

Wie oben schon beschrieben wurde, sind Polyvinylalkohole mit entsprechend hohem Hydrolysierungsgrad nur unter besonderen Bedingungen direkt tablettierbar, d. h. es müssen vorab Granulierungsschritte durchgeführt werden oder die eingesetzten PVAs müssen mit weiteren Tablettierhilfmitteln und leicht komprimierbaren Bindemitteln versetzt werden, so dass der Anteil an Polyvinylalkohol in der Gesamtzusammensetzung verringert ist.

Durch Versuche wurde nun gefunden, dass für die gute Verarbeitbarkeit in Tablettenformulierungen nicht nur der Hydrolysegrad der eingesetzten Polyvinylalkohole, und damit die Kristallinität, eine Rolle spielt, sondern auch die physikalischen Eigenschaften und Erscheinungsformen der eingesetzten handelsüblichen PVA-Qualitäten.

Überraschender Weise wurde gefunden, dass die Partikelgröße der verwendeten PVA-Qualitäten offenbar einen Einfluß auf die Tablettierbarkeit hat. In diesem Zusammenhang wurde weiterhin gefunden, dass abhängig von der mittleren Partikelgröße der PVA-Pulver direkt tablettierbare Mischungen hergestellt werden können, worin der Gehalt an PVAs mehr als 60 % betragen kann.

Eine Lösung dieses Problems besteht also darin, dass ein handelsübliches, pulverförmiges Polyvinylalkohol in geeigneter Weise mit einer sehr gut komprimierbaren Komponente kombiniert wird, wodurch ein direkt verpressbares, pulverförmiges Produkt erhalten wird, das vorwiegend aus dem eingesetzten PVA besteht. Im Folgenden ist es möglich, durch einfaches Vermischen des erfindungsgemäßen Produkts mit einem gewünschten Wirkstoff ohne weiter Behandlung und Verpressen mit geeignetem Druck Tabletten herzustellen. Gegebenenfalls können vor dem Verpressen der Mischung wenige weitere Zuschlagstoffe, wie z.B. Schmiermittel und andere Additive zugesetzt werden. Wesentlich ist, dass es keiner weiteren Behandlung bedarf, um das erhaltene Pulvergemisch zu Tabletten verpressen zu können.

Überraschender Weise wurde durch Versuche gefunden, dass man die verschiedensten Polyvinylalkohole in eine direkt verpressbare Tablettiermatrix überführen kann, wenn man gemahlene, pulverförmige PVAs mit mikrokristallinen Cellulosen (MCC) versetzt. Als besonders überraschend hat sich in diesem Zusammenhang gezeigt, dass für die Erzielung solcher Direktverpressungseigenschaften ganz offensichtlich nur die MCCs geeignet sind; andere üblicherweise die Direktverpressung fördernde Träger, wie z.B. direkt verpessbare Calciumhydrogenphosphate, einschließlich des per se sehr gut direkt tablettierbare Fujicalin®, direkt verpressbare Sorbitole (z.B. Parteck® SI 400), direkt verpressbare Mannitole (z.B. Parteck® M200) oder direkt verpressbare Stärken (z.B. Starch 1500), zeigen in der Kombination mit PVAs nicht diese Wirkung und führen nicht zu direkt verpressbaren Pulvermischungen mit den PVAs, wie die eigenen Untersuchungen gezeigt haben.

Durch diesen überraschender Weise gefundenen Effekt kann dem Galeniker nun eine vorwiegend aus PVA bestehende, direkt verpressbare Vormischung für die Tablettenherstellung zur Verfügung gestellt werden, die zur Beschleunigung eines Entwicklungsprozesses einer neuen Tablettenformulierung führt.

Microcrystalline Cellulose (MCC) ist ein Tablettierhilfsmittel in der Pharmazeutikaherstellung und wird bevorzugt als Wirkstoffträger eingesetzt und ist eine Komponente für fast jede Art von oralen Dosierungsformen, wie Tabletten, Kapseln, Sachets, Granulate und andere.

In Reinform ist Mikrokristalline Cellulose (MCC) mit der allgemeinen Formel $(C_6H_{10}O_5)_n$ weiße, freifließende Cellulose in Pulverform, die im Handel mit unterschiedlicher Körnung erhältlich ist. In pharmazeutischer Qualität erfüllt sie die Standards der üblichen Arzneibücher wie z.B. der Ph. Eur., der USP/NF oder der JP. Mikrokristalline Cellulose dient unter anderem als unverdaulicher, nicht resorbierbarer Ballaststoff für kalorienreduzierte Lebensmittel, etwa Salatsoßen, Desserts und Eiscremes, als Trennmittel oder als Trägerstoff. Wie aus der vorhergehenden Beschreibung zu entnehmen ist, dient sie in der Pharmazie als Bindemittel oder Trägerstoff für die Tablettenherstellung. In diesem Zusammenhang hat sie sich als geeignet für die Direkttablettierung erwiesen und führt zu harten Tabletten, die bei geeigneter Formulierung kurze Zerfallszeiten aufweisen.

MCC wird aus verholzten Pflanzenteilen gewonnen (nicht aus Altpapier). Hierbei wird die Pflanzen-Zellulose mit verdünnter Salzsäure bei Temperaturen über 100° C von nichtkristallinen Zellulose-Anteilen befreit. Das heißt, MCC in Pharmaqualität lässt sich durch partielle Hydrolyse von hoch reiner Cellulose und anschließender Aufreinigung und Trocknung gewinnen. Im Anschluss an die Hydrolyse kann wahlweise eine Carboxylierung erfolgen, um die hydrophilen Eigenschaften zu verbessern.

MCC ist in Wasser, Alkoholen und organischen Lösungsmitteln unlöslich. In Wasser bildet MCC eine dreidimensionale Matrix, bestehend aus unzähligen, unlöslichen Mikrokristallen, die ein stabiles thixotropes Gel ausbilden. Die vorteilhaften Eigenschaften von MCC bleiben auch erhalten bei Temperatur bedingten Änderungen des Phasenzustands, beispielsweise beim Übergang in den Gefrierzustand oder beim Erhitzen auf höhere Temperaturen, so dass MCC gut geeignet ist für Fertigmischungen zur Weiterverarbeitung.

Als geeignete MCCs zur Erzielung ausreichenden Tablettenhärten haben sich die handelsübliche Typen erwiesen, die mittlere Partikelgrößen $D_{v50}$ im Bereich von 60 bis 250 $\mu$m, bevorzugt im Bereich von 80 bis 200 $\mu$m, besonders bevorzugt im Bereich von 80 bis 150 $\mu$m, ganz besonders bevorzugt im Bereich von 90 bis 140 $\mu$m, besitzen,

ermittelt durch Laserdiffraktionsbestimmung. Dabei weisen solche MCC-Typen vorzugsweise Schüttdichten im Bereich von 0,22 bis 0,38 g/cm$^3$, bevorzugt im Bereich von 0,24 bis 0,35 g/cm$^3$, besonders bevorzugt im Bereich von 0,28 bis 0,33 g/cm$^3$ auf. Geeignete handelsübliche MCC-Qualitäten, die diese Kriterien erfüllen und für die Verwendung in pharmazeutischen Formulierungen qualifiziert sind, sind beispielsweise Vivapur 102 (im Luftstrom getrocknet, mittlere Partikelgröße von etwa 100 μm, ermittelt durch Laserdiffraktion, Schüttdichte 0,28 - 0,33 g/cm$^3$), Avicel PH 102 (mittlere Partikelgröße etwa 100 μm, Schüttdichte 0,28 - 0,33 g/cm$^3$) sowie Emcocel 90M (Sprühgetrocknet, mittlere Partikelgröße von etwa 100 μm, ermittelt durch Laserdiffraktion, Schüttdichte 0,25 - 0,37 g/cm$^3$).

Aber auch andere nicht hier erwähnte Handelsprodukte, die die beschriebenen Erfordernisse erfüllen, können gemäß der hier beschriebenen Erfindung verwendet werden.

Besonders überraschend ist, dass durch Zusatz geeigneter mikrokristalliner Cellulosen zu den verschiedensten PVA-Qualitäten, insbesondere zu PVAs mit unterschiedlichsten Viskositäten, direkt verpressbare Mischungen erhalten werden, die vorwiegend aus PVAs bestehen.

Es hat sich als besonders vorteilhaft erwiesen, wenn in den erfindungsgemäßen Zusammensetzungen das Verhältnis der beschriebenen PVAs und MCCs in einem Bereich 2 : 1 bis 1 : 2 bezogen auf das Gewicht, bevorzugt in einem Verhältnis in einem Bereich von 2 : 1 bis 1:1 liegt. Solche Co-Mischungen haben sich als besonders geeignet erwiesen zur Herstellung von Tabletten mit verzögerter Wirkstofffreisetzung. Nach intensiver Vermischung weisen die hier gefundenen Co-Mischungen aus PVA mit MCCs Schüttdichten im Bereich von 0,43 - 0,45 g/ml bei Stampfdichten im Bereich von 0,58-0,60 g/ml auf.

Durch die beschriebenen vorteilhaften Eigenschaften der Kombinationen aus PVA und MCC erhält der Formulierer in der pharmazeutischen Industrie, aber auch in der Lebensmittelindustrie oder in anderen technischen Bereichen ein Material an die Hand, welches den Entwicklungsaufwand für feste komprimierte Darreichungsformen mit verlängerter Wirkstofffreisetzung deutlich vereinfacht. Er braucht nur noch seinen zu retardierenden Wirkstoff mit der PVA-MCC-Kombination zu mischen, ggf. einige wenige Hilfsstoffe insbes. Gleit-und Schmiermittel zugeben und diese Mischung dann auf einer Tablettiermaschine verpressen. Durch die besonders guten Tablettiereigenschaften dieser Matrix ist die Entwicklung von Retardtabletten auch mit Wirkstoffen möglich geworden, die per se eigentlich als nicht direkt verpressbar gelten und die in üblicher Weise durchgeführten Prozessen granuliert werden müssten. Die Verwendung der PVA-MCC-Matrix spart Entwicklungszeit, Geräteinvestitionen und führt zu einer verbesserten Prozesssicherheit in Entwicklung und Produktion.

Ein vorteilhafter Nebeneffekt stellt sich bei Verwendung der erfindungsgemäßen Co-Mischungen im Tablettierungsprozess ein, der darin besteht dass die erfindungsgemäßen Mischungen zu vergleichsweise niedrigen Ausstoßkräften führen, wodurch es möglich ist, mit deutlich geringeren Mengen an Schmiermitteln zu arbeiten als sonst in der Tablettierung üblich. So werden statt eines üblicherweise 1%igen Magnesiumstearatzusatzes nur noch etwa ein Viertel dieser Menge benötigt, gegebenenfalls auch noch weniger. Unter besonderen Bedingungen kann auch auf den Zusatz von solchen Schmiermitteln gänzlich verzichtet werden. Dies bedingt eine zusätzliche Verbesserung der interpartikulären Bindungskräfte, d.h. es werden härtere Tabletten bei gleicher Presskraft erhalten, wobei eine reproduzierbarere Wirkstofffreisetzung erzielt werden kann. Letzteres ist dadurch begründet, dass die Freisetzung im Wesentlichen über den PVA-Gehalt gesteuert wird und der reduzierte Zusatz von hydrophobem Magnesiumstearat nur noch einen geringfügigen Einfluss auf das Freisetzungsverhalten ausübt.

Des Weiteren betrifft die vorliegende Erfindung ein Verfahren zur Beeinflussung der Tablettiereigenschaften von feinkörnigen PVA-Qualitäten, insbesondere von gemahlenen PVAs, die an sich nur eine geringe Kompressibiliäten aufweisen. Durch Versuche wurde gefunden, dass diese PVAs durch Kombination mit MCCs in eine direkt verpessbare Form überführt werden können.

Feinkörnige PVAs sind als Retardierungsmatrices besonders geeignet, da sie in der Regel sehr gut verarbeitet werden können, um homogenere Mischungen mit dem zu retardierenden Wirkstoff herzustellen. Letzteres ist für die Einzeldosierungsgenauigkeit "Content Uniformity" von besonderer Bedeutung, um in jeder Einzeltablette immer die gleiche Wirkstoffmenge zu erhalten.

Außerdem hat diese Art der Formulierung mit feinkörnigen PVA-Qualitäten den Vorteil, dass die großen Oberflächen der feinen PVA-Partikel zu einer homogeneren Gelschichtbildung nach der Befeuchtung im Magen-DarmTrakt führt, was bei Einnahme der Tabletten durch den Patienten zu einer reproduzierbareren und ggf. auch verlängerten Diffusion des Wirkstoffs durch dieses Gel führt.

Durchführung

Zur Herstellung der erfindungsgemäßen Co-Mischungen werden geeignete feinkörnig gemahlene Polyvinylalkohole (PVAs) mit mikrokristallinen Cellulosen (MCCs) intensiv vermischt und so in Co-Mischungen überführt, die als direkt verpressbare Tablettiermatrices hervorragend geeignet sind. Dieses ist besonders überraschend, da Abmischungen solcher PVAs mit anderen -per se auch sehr gut verpressbaren- direkt tablettierbaren Hilfsstoffen des Marktes diesen Direktverpressungseffekt mit den pulverförmigen PVAs nicht zeigen. In den folgenden Versuchen kann anhand einer Formulierung mit pulverförmiger Ascorbinsäure als Modellwirkstoff gezeigt werden, dass solchermaßen hergestellte PVA-MCC Co-Mischungen sich sehr gut zur Direktverpressung von schlecht verpressbaren Wirkstoffen eignen. Weiterhin kann mit den hergestellten Tabletten, die entsprechende Co-Mischungen als Wirkstoffträger enthalten, gezeigt werden, dass aus solchermaßen erzeugten Tabletten der Wirkstoff über sehr lange Zeit kontrolliert freigesetzt werden kann. Die durchgeführten Versuche haben gezeigt, dass entsprechende wirkstoffhaltige Tabletten verzögerte Wirkstofffreisetzungen von mindestens 2 Stunden, bevorzugt über mindestens 6 Stunden, besonders bevorzugte von mindestens 8 Stunden, insbesondere bevorzugt von mindestens 10 Stunden, und ganz besonders bevorzugt von bis zu 12 Stunden aufweisen, abhängig vom eingesetzten Wirkstoff und vom Mischungsverhältnis der Polyvinylalkohole und der mikrokristallinen Cellulosen.

Da im Zusammenhang mit der Herstellung von Tablettenformulierungen der Begriff "direkt verpressbar" nicht verbindlich definiert ist wird in der vorliegenden Beschreibung das Pressverhalten eines sehr gut direkt verpressbaren Mannits des Marktes (Parteck® M 200 (Mannitol)), geeignet für die Verwendung als Excipient EMPROVE® exp Ph Eur, BP, JP, USP, E 421, Artikel Nr. 1.00419, Merck KGaA, Darmstadt, Deutschland) als Maßstab zum Vergleich verwendet. Ziel ist es mit den direkt verpressbaren Co-Mischungen, die PVAs in größerer Menge enthalten, insbesondere hinsichtlich ihrer Kompressibiliät dem Verhalten des Parteck® M 200 möglichst nahe zu kommen.

Durch die durchgeführten Versuche wurde gefunden, dass wirkstoffhaltige Tabletten, die eine erfindungsgemäße Zusammensetzung in Form einer Co-Mischung in einer Menge von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette enthalten, die gewünschte, verlängerte Wirkstofffreisetzung aufweisen. Vorteilhafterweise können mit solchen Zusammensetzungen wie gewünscht bereits bei Einsatz von niedrigen Preßkräften Tabletten mit besonders hohen Tablettenhärten erhalten werden, die im Herstellungsprozess überraschend geringe Ausstoßkräfte erfordern. Wie durch Versuche zur Herstellung von Placebos gefunden wurde, werden bereits bei Einsatz einer Presskraft von 19,5 kN Tabletten mit einer Tablettenhärte von 295,7 N erhalten, die nur eine Ausstoßkraft von etwa 66,7 N erfordern. Darüber hinaus weisen diese Tabletten nur geringe Friabilitäten auf, wie durch geeignete Versuche gezeigt werden kann.

Durch die vorliegende Erfindung wird somit ein Verfahren zur Herstellung von direkt verpressbaren Zusammensetzungen mit verlängerter Wirkstofffreisetzung zur Verfügung gestellt, wodurch eine Co-Mischung aus mikrokristallinen Cellulosen (MCCs) und Polyvinylalkoholen (PVAs) hergestellt wird, worin Polyvinylalkohol zu einem feinkörnigen Pulver mit einer mittleren Partikelgröße $D_{v50}$ im Bereich von 50 bis 260 $\mu$m, einer Schüttdichte im Bereich von 0,55 bis 0,62 g/ml, einer Stampfdichte im Bereich von 0,72 bis 0,85 g/ml und einem Schüttwinkel im Bereich von 35 bis 38° gemahlen und durch ein 800 $\mu$m Sieb gesiebt wird, und das erhaltene Pulver mit microkristalliner Cellulose (MCCs) mit mittlerer Partikelgröße $D_{v50}$ im Bereich von 60 bis 250 $\mu$m, und einer Schüttdichte im Bereich von 0,22 bis 0,38 g/cm$^3$ intensiv vermischt wird. Auf diese Weise wird eine direkt verpressbare Co-Mischung mit einer Schüttdichte im Bereich von 0,40 bis 0,48 g/ml, einer Stampfdichte im Bereich von 0,55 bis 0,63 g/ml und einem Schüttwinkel im Bereich von 35 bis 38° erhalten, die je nach Wunsch mit unterschiedlichen Wirkstoffen versetzt werden und zu Tabletten mit verzögerter Wirkstofffreisetzung verpresst werden kann.

Die im Folgenden gegebenen Beispiele offenbaren Methoden und Bedingungen zur Herstellung erfindungsgemäßer PVA-MCC-Co-Mischungen. Für den Fachmann auf dem Gebiet ist es selbsterklärend, dass auch andere Methoden zum Vermahlen und zum Vermischen der Ausgangssubstanzen zur Verfügung stehen als hier beschrieben sind.

Aus den Beispielen ergeben sich die besonderen Vorteile dieser PVA-MCC-Kombinationen im Vergleich zu den ungenügenden Kompressibilitäten die durch PVA-Kombinationen mit anderen - aber als besonders gut tablettierbar geltenden - Trägerstoffen erhalten werden.

Bei Abmischung einer erfindungsgemäßen PVA-MCC-Matrix mit einer per se schlecht verpressbaren pulverförmigen Ascorbinsäure (als Modellwirkstoff) und Zugabe einer sehr geringen Menge Magnesiumstearat als Schmiermittel können durch einfache Direkttablettierung Tabletten ausreichender Härten mit geringem mechanischem Abrieb erhalten werden, welche problemlos zur weiteren Behandlung z.B. zur Abfüllung in Blisterverpackungen oder zur bruchfreien Entnahme aus diesen Durchdrückpackungen durch den Patienten zur Verfügung stehen. Entsprechende Ascorbinsäure-haltige Tabletten zeigen, dass eine verlängerte Ascorbinsäurefreisetzung aus solchen PVA-MCC-Matrixtabletten über mehrere Stunden gewährleistet werden kann.

Liste der Abbildungen:

Fig. 1:    In **Figur 1** sind die Presskraft-Härte-Profile gemäß der Daten aus Tabelle 1 graphisch dargestellt.

Fig. 2:    In **Figur 2** sind die Presskraft-Abrieb-Profile gemäß der Daten aus Tabelle 1 graphisch dargestellt.

**Fig. 3:** In **Figur 3** sind die Presskraft-Härte-Profile der Daten gemäß der Zusammensetzungen aus Tabelle 4 dargestellt.

**Fig. 4:** In **Figur 4** sind die Presskraft-Abrieb-Profile graphisch anhand der Daten aus Tabelle 4 dargestellt.

**Fig. 5:** In **Figur 5** ist die Freisetzung von Ascorbinsäure aus Retardtabletten gemäß Muster 1, charakterisiert durch Daten aus Tabelle 9, graphisch dargestellt.

**Fig. 6:** In Figur 6 ist die Freisetzung von Ascorbinsäure aus Retardtabletten (Muster 2) charakterisiert durch Daten aus Tabelle 9, graphisch dargestellt.

<u>Beispiele</u>

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung.

Zum besseren Verständnis und zur Verdeutlichung der Erfindung werden im Folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew.- bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten in °C.

**Geräte/Verfahren zur Charakterisierung der Stoffeigenschaften**

1. <u>Schüttdichte</u>: gemäß DIN EN ISO 60: 1999 (Deutsche Fassung) -Angabe in "glmi"

2. <u>Stampfdichte</u>: gemäß DIN EN ISO 787-11: 1995 (Deutsche Fassung) -Angabe in "g/ml"

3. <u>Schüttwinkel</u>: gemäß DIN ISO 4324: 1983 (Deutsche Fassung) -Angabe in "Grad"

4. <u>Oberfläche</u> bestimmt gemäß BET: Auswertung und Durchführung gemäß Literatur "BET Surface Area by Nitrogen Absorption" von S. Brunauer et al. (Journal of American Chemical Society, 60, 9, 1983) Gerät: ASAP 2420 Fa. Micromeritics Instrument Corporation (USA); Stickstoff; Einwaage: ca. 3,0000 g; Ausheizung: 50°C (5 h); Heizrate 3 K/min; Angabe des arithmetischen Mittelwertes aus drei Bestimmungen

5. <u>Partikelgrößenbestimmung über Laserbeugung mit Trockendispergierung</u>: Mastersizer 2000 mit Dispergiereinheit Scirocco 2000 (Fa. Malvern Instruments Ltd. UK), Bestimmungen bei 1, 2 und 3 bar Gegendruck; Auswertung Fraunhofer; Dispersant RI: 1.000, Obscuration Limits: 0.1-10.0%, Tray Type: General Purpose, Background Time: 7500 msec, Measurement Time: 7500 msec, Durchführung gemäß ISO 13320-1 sowie den Angaben des technischen Handbuchs und den Spezifikationen des Geräteherstellers; Angaben in Vol%

6. <u>Partikelgrößenbestimmung über Laserbeugung mit Naßdispergierung</u>: Mastersizer 2000 mit Naßdispergiereinheit Hydro 2000SM (Fa. Malvern Instruments Ltd. UK); Dispersionsmedium Silikonöl dünnflüssig (Hersteller: Evonik Goldschmidt GmbH, Deutschland; Bezeichnung des Herstellers: Tegiloxan3, Artikelnummer des Herstellers: 9000305); Dispersant RI: 1.403; Stirrer Speed: 2500 rpm; Tray Type: General Purpose; Background Time: 7500 msec; Measurement Time: 7500 msec; Obscuration Limits: 7.0-13.0%; Durchführung gemäß ISO 13320-1 sowie den Angaben des technischen Handbuchs und den Spezifikationen des Geräteherstellers; Angaben in Vol.-%. Durchführung: Die Suspensionszelle wird mit dem dünnflüssigen Silikonöl gefüllt, portionsweise die Probe bis zum Erreichen des angestrebten Obscurationbereichs (7,0-13,0%) zugegeben und nach 2 Minuten Wartezeit die Messung gestartet.

7. Partikelgrößenbestimmung durch Trockensiebung über einen Siebturm: Retsch AS 200 control, Fa.Retsch (Deutschland); Substanzmenge: ca. 110,00 g; Siebzeit: 30 Minuten; Amplitudenintensität: 1 mm; Intervall: 5 Sekunden; Analysensiebe mit Metalldrahtgewebe gemäß DIN ISO 3310; Siebweiten (in $\mu$m): 710, 600, 500, 400, 355, 300, 250, 200, 150, 100, 75, 50, 32; Angabe der Mengenverteilung pro Siebfraktion in den Tabellen als "Gew.% der Einwaage"

8. Die Tablettierungsprüfungen erfolgen folgendermaßen:
Die Mischungen gemäß der im Versuchsteil angegebenen Zusammensetzungen werden 5 Minuten in einem verschlossenen Edelstahlbehälter (Fassungsvolumen: ca. 2 l, Höhe: ca. 19,5 cm, Durchmesser: ca. 12 cm Außenmaß) auf einem Labor-Taumelmischer (Turbula T2A, Fa.Willy A. Bachofen, Schweiz) gemischt.
Als Magnesiumstearat wird Parteck LUB MST (Magnesiumstearat pflanzlich) EMPROVE exp Ph Eur, BP, JP, NF, FCC Artikel Nr. 1.00663 (Merck KGaA, Deutschland) eingesetzt welches über ein 250 $\mu$m Sieb abgelegt wurde.
Die Verpressung zu 500 mg Tabletten (11 mm Stempel, rund, flach, mit Facette) erfolgt auf einer instrumentierten Excenter-Tablettiermaschine Korsch EK 0-DMS (Fa.Korsch, Deutschland) mit dem Auswertesystem Catman 5.0 (Fa.Hottinger Baldwin Messtechnik - HBM, Deutschland).
Je getesteter Presskraft (Soll-Einstellungen: ~5, -10, ~20 und ~30 kN; die effektiv gemessenen Ist-Presskräfte sind in den Beispielen angegeben) werden mindestens 100 Tabletten zur Auswertung der Pressdaten und der galenischen Kennzahlen hergestellt.
Tablettenhärten, Durchmesser und Höhen: Erweka Multicheck 5.1 (Fa. Erweka, Deutschland); Durchschnittsdaten (arithmetische Mittelwerte) aus jeweils 20 Tablettenmessungen pro Presskraft. Die Messungen erfolgen einen Tag nach der Tablettenherstellung.
Tablettenabrieb: Friabilitätsprüfgerät TA420 (Fa. Erweka, Deutschland); Geräteparameter und Ausführung der Messungen gemäß Ph.Eur. 7. Ausgabe "Friabilität von nicht überzogenen Tabletten". Die Messungen erfolgen einen Tag nach Tablettenherstellung.
Tablettenmasse: Multicheck 5.1 (Fa. Erweka, Deutschland) mit der Waage Sartorius CPA 64 (Fa. Sartorius, Deutschland). Angabe des Durchschnittswerts (arithmetischer Mittelwert) aus der Wägung von 20 Tabletten je Presskraft. Die Messungen erfolgen einen Tag nach Tablettenherstellung.

9. Ascorbinsäure Freisetzungsprüfung: Die Ascorbinsäure enthaltenden Komprimate (aus den Verpressungen mit 20kN Presskraft) werden in einer in-vitro Freisetzungsapparatur der Firma SOTAX (Allschwil, Schweiz) mit dem in der USP 36 unter <711> beschriebenen "Apparatus 2 (Paddle Apparatus)" und unter den dort beschriebenen Bedingungen für "Extended-release dosage forms" vermessen (USP=United States Pharmacopoeia). Die Probenziehung erfolgt automatisch über ein Schlauchpumpensystem mit anschl. Vermessung in einem Spectrometer 8453 (Agilent Technologies, USA) und einer Durchflussküvette.
Die gemittelten Werte ergeben sich aus den Freisetzungsprüfungen von jeweils 6 Ascorbinsäure haltigen Tabletten gepresst mit 20 kN Presskraft.

Verwendete Ascorbinsäure zur Tablettierung: L(+)-Ascorbinsäure, Ph Eur, USP, NF, Product 83568.290 (VWR, Deutschland)

Messapparaturen und Messparameter:

1.- Freisetzungsapparatur Sotax AT7s ausgestattet mit Apparatus 2 (Paddle Apparatus gemäß USP 36)

- Temperatur: 37°C +/- 0,5°C
- Drehzahl der Paddle: 100 Upm
- Volumen Freisetzungsmedium je Messgefäß: 900 ml
- Tablettenmasse: 500 mg
- Gesamtlaufzeit der Messung: 720 min. (mit Probenziehung nach 15, 30, 45, 60, 120, 180, 240, 300, 360, 420, 480, 540, 600, 660, 720 min.)

2.- Schlauchpumpe zur Probenziehung: Sotax CY 7-50 (Fa. SOTAX, Schweiz)

3.- Spectrometer 8453 (Agilent Technologies, USA)

- Messung bei 244 nm in einer 2mm Durchflußmesszelle
- Auswertung über Excel
- Aufbereitung Medium: Dosa Prep X8 (Fa. DOSATEC GmbH, Deutschland)

Zusammensetzung (in Gew.%) des Freisetzungsmediums:

| | |
|---|---|
| Kaliumdihydrogenphosphat (Artikel Nr. 1.04873, Merck KGaA Darmstadt, Deutschland) | 0,68% |
| Titriplex III (Artikel Nr. 1.08418, Merck KGaA, Darmstadt, Deutschland) | 0,02% |
| Phosphorsäure 85%, (Artikel Nr. 1.00573, Merck KGaA Darmstadt, Deutschland) | 0,20% |
| Vollentsalztes Wasser | 99,10% |

**Charakterisierung der verwendeten Materialien**

**1. Verwendete PVA-Typen und ihre Eigenschaften:**

**1.1 Rohstoffe zur Mahlung**

1.1.1. PVA 4-88: Polyvinylalkohol 4-88, geeignet für die Verwendung als Excipient EMPROVE® exp Ph Eur, USP, JPE, Artikel Nr. 1.41350, Merck KGaA, Darmstadt, Deutschland

1.1.2. PVA 18-88: Polyvinylalkohol 18-88, geeignet für die Verwendung als Excipient EMPROVE® exp Ph Eur, USP, JPE, Artikel Nr. 1.41355, Merck KGaA, Darmstadt, Deutschland

1.1.3. PVA 26-88: Polyvinylalkohol 26-88, geeignet für die Verwendung als Excipient EMPROVE® exp Ph Eur, USP, JPE, Artikel Nr. 1.41352, Merck KGaA, Darmstadt, Deutschland

1.1.4. PVA 40-88: Polyvinylalkohol 40-88, geeignet für die Verwendung als Excipient EMPROVE® exp Ph Eur, USP, JPE, Artikel Nr. 1.41353, Merck KGaA, Darmstadt, Deutschland

1.1.5. PVA 28-99: Polyvinylalkohol 28-99, geeignet für die Verwendung als Excipient EMPROVE® exp JPE, Artikel Nr. 1.41356, Merck KGaA, Darmstadt, Deutschland

Diese PVA-Typen liegen als grobkörnige, mehrere Millimeter große Partikel vor, die in dieser Form nicht als eine direkt verpressbare Tablettiermatrix einsetzbar sind.

Die großen Partikel erlauben keine reproduzierbare Befüllung der Stempelmatrizen und somit auch kein konstantes Tablettengewicht bei hohen Rotationsgeschwindigkeiten der (Rundlauf) Tablettiermaschinen. Außerdem können nur feinkörnige PVAs eine homogene Verteilung des Wirkstoffes, ohne das Auftreten von Entmischungseffekten, in der Tablette gewährleisten; dies ist für die Sicherstellung der Einzeldosiergenauigkeit des Wirkstoffes (content uniformity) in jeder produzierten Tablette unbedingt notwendig. Zusätzlich kann nur durch ein feinkörniges PVA auch die für die reproduzierbare Retardierung notwendige homogene Gelbildung im gesamten Tablettenkörper gewährleistet werden.

Aus diesen Gründen müssen die o.g. grobkörnigen PVA-Typen vor ihrer Verwendung als direkt verpressbare Retardierungsmatrices zerkleinert d.h. gemahlen werden.

**1.2 Gemahlene PVA-Typen**

1.2.1. Gemahlenes PVA 4-88, aus Polyvinylalkohol 4-88 Artikel Nr. 1.41350, Merck KGaA, Darmstadt, Deutschland

1.2.2. Gemahlenes PVA 18-88, aus Polyvinylalkohol 18-88 Artikel Nr. 1.41355, Merck KGaA, Darmstadt, Deutschland

1.2.3. Gemahlenes PVA 26-88, aus Polyvinylalkohol 26-88 Artikel Nr. 1.41352, Merck KGaA, Darmstadt, Deutschland

1.2.4. Gemahlenes PVA 40-88, aus Polyvinylalkohol 40-88 Artikel Nr. 1.41353, Merck KGaA, Darmstadt, Deutschland

1.2.5. Gemahlenes PVA 28-99, aus Polyvinylalkohol 28-99 Artikel Nr. 1.41356, Merck KGaA, Darmstadt, Deutsch-

land

<u>Vermahlung:</u>

Die Vermahlung der PVA-Typen erfolgt auf einer Aeroplex Spiralstrahlmühle Typ 200 AS der Firma Hosokawa Alpine, Augsburg, Deutschland unter flüssigem Stickstoff als Kaltvermahlung im Temperaturbereich von 0°C bis minus 30°C,

Die resultierenden Produkteigenschaften der gemahlenen PVA-Typen insbes. die Pulverkennzahlen wie Schüttdichte, Stampfdichte, Schüttwinkel, BET-Oberfäche, BET-Porenvolumen sowie die Partikelgrößenverteilungen ergeben sich aus den nachfolgenden Tabellen:

<u>Schüttdichte. Stampfdichte. Schüttwinkel, BET-Oberfläche, BET-Porenvolumen:</u>

(Details zu den Messverfahren siehe unter Methoden)

| Muster | Schüttdichte (g/ml) | Stampfdichte (g/ml) | Schüttwinkel (°) | BET-Oberfläche ($m^2$/g) | BET-Porenvolumen ($cm^3$/g) |
|---|---|---|---|---|---|
| **PVA 4-88\*** | 0,61 | 0,82 | 35,1 | 0,1308 | 0,0008 |
| **PVA 18-88\*** | 0,57 | 0,76 | 35,5 | 0,1831 | 0,0011 |
| **PVA 26-88\*** | 0,56 | 0,74 | 35,5 | 0,2045 | 0,0013 |
| **PVA 40-88\*** | 0,59 | 0,77 | 36,9 | 0,1123 | 0,0009 |
| **PVA 28-99\*** | 0,58 | 0,76 | 37,7 | 0,2210 | 0,0016 |
| * vermahlenes PVA | | | | | |

<u>Partikelverteilung bestimmt über Laserbeugung mit Trockendispergierung (1 bar Gegendruck):</u>

Angaben in $\mu$m (Details zum Messverfahren siehe unter Methoden)

| Muster PVA | Dv5 | Dv10 | Dv20 | Dv25 | Dv30 | Dv50 | Dv75 | Dv90 |
|---|---|---|---|---|---|---|---|---|
| **4-88\*** | 21,36 | 33,93 | 60,39 | 75,25 | 91,61 | 177,74 | 380,57 | 790,37 |
| **18-88\*** | 29,67 | 44,93 | 73,95 | 89,11 | 105,22 | 185,49 | 375,88 | 755,84 |
| **26-88\*** | 27,76 | 42,32 | 73,01 | 90,14 | 108,67 | 198,51 | 382,65 | 676,96 |
| **40-88\*** | 31,84 | 50,64 | 89,13 | 109,77 | 131,45 | 230,52 | 413,71 | 634,59 |
| **28-99\*** | 24,87 | 39,81 | 72,81 | 90,72 | 109,31 | 191,42 | 343,54 | 561,23 |
| * vermahlenes PVA | | | | | | | | |

<u>Partikelverteilung bestimmt über Laserbeugung mit Trockendispergierung(2 bar Gegendruck):</u>

Angaben in μm (Details zum Messverfahren siehe unter Methoden)

| Muster PVA | Dv5 | Dv10 | Dv20 | Dv25 | Dv30 | Dv50 | Dv75 | Dv90 |
|---|---|---|---|---|---|---|---|---|
| 4-88* | 19,09 | 30,21 | 52,69 | 64,83 | 77,87 | 143,83 | 279,64 | 451,94 |
| 18-88* | 26,90 | 40,38 | 65,30 | 78,08 | 91,55 | 159,10 | 321,46 | 607,64 |
| 26-88* | 24,59 | 36,93 | 61,67 | 75,05 | 89,33 | 157,79 | 286,17 | 434,23 |
| 40-88* | 31,03 | 49,47 | 88,54 | 110,06 | 132,79 | 235,87 | 430,35 | 686,10 |
| 28-99* | 24,27 | 39,63 | 74,31 | 93,13 | 112,51 | 196,45 | 350,21 | 570,12 |
| * vermahlenes PVA | | | | | | | | |

Partikelverteilung bestimmt über Laserbeugung mit Trockendispergierung (3 bar Gegendruck):

Angaben in μm (Details zum Messverfahren siehe unter Methoden)

| Muster PVA | Dv5 | Dv10 | Dv20 | Dv25 | Dv30 | Dv50 | Dv75 | Dv90 |
|---|---|---|---|---|---|---|---|---|
| 4-88* | 18,35 | 29,27 | 51,25 | 63,09 | 75,77 | 139,46 | 269,80 | 425,62 |
| 18-88* | 24,55 | 36,60 | 57,91 | 68,48 | 79,45 | 132,37 | 246,56 | 393,59 |
| 26-88* | 25,17 | 38,18 | 64,35 | 78,47 | 93,57 | 167,41 | 317,16 | 514,18 |
| 40-88* | 32,81 | 53,33 | 96,27 | 119,6 1 | 144,21 | 256,31 | 463,67 | 717,76 |
| 28-99* | 22,33 | 35,92 | 65,94 | 82,31 | 99,37 | 174,84 | 305,50 | 454,03 |
| * vermahlenes PVA | | | | | | | | |

Partikelverteilung bestimmt über Laserbeugung mit Nassdispergierung- (in dünnflüssigem Silikonöl):

Angaben in μm (Details zum Messverfahren siehe unter Methoden)

| Muster PVA | Dv5 | Dv10 | Dv20 | Dv25 | Dv30 | Dv50 | Dv75 | Dv90 |
|---|---|---|---|---|---|---|---|---|
| 4-88* | 10,03 | 20,10 | 38,02 | 47,82 | 58,31 | 110,91 | 231,64 | 390,95 |
| 18-88* | 17,19 | 30,25 | 50,06 | 59,22 | 68,47 | 111,89 | 212,70 | 357,70 |
| 26-88* | 15,42 | 26,76 | 45,50 | 54,83 | 64,47 | 110,50 | 212,91 | 353,68 |
| 40-88* | 20,41 | 34,80 | 60,35 | 73,32 | 86,96 | 154,96 | 299,57 | 490,08 |
| 28-99* | 14,68 | 25,96 | 47,49 | 58,88 | 70,80 | 127,68 | 240,70 | 376,70 |
| * vermahlenes PVA | | | | | | | | |

Partikelverteilung bestimmt über Turmsiebung:

| Muster PVA | 300-355 µm | 355-400 µm | 400-500 µm | 500-600 µm | 600-710 µm | >710 µm |
|---|---|---|---|---|---|---|
| 4-88* | 4,5 | 2,8 | 3,5 | 2,0 | 0,9 | 0,8 |
| 18-88* | 4,2 | 2,6 | 3,5 | 2,1 | 1,0 | 1,0 |
| 26-88* | 3,7 | 2,2 | 2,7 | 1,8 | 0,6 | 0,5 |
| 40-88* | 6,6 | 3,9 | 5,9 | 4,1 | 1,9 | 2,6 |
| 28-99* | 5,3 | 3,2 | 3,7 | 2,0 | 0,8 | 0,8 |

* vermahlenes PVA

## 2. Direkt verpressbare Trägerstoffe zur Herstellung der Abmischungen mit Polyvinylalkoholen (vermahlen)

2.1 Parteck® SI 150 (Sorbit), geeignet für die Verwendung als Excipient EMPROVE® exp Ph Eur, BP, JP, JSFA, NF, E 420, Artikel Nr. 1.03583, Merck KGaA, Darmstadt, Deutschland
2.2 Parteck® M 200 (Mannitol), geeignet für die Verwendung als Excipient EMPROVE® exp Ph Eur, BP, JP, USP, E 421, Artikel Nr. 1.00419, Merck KGaA, Darmstadt, Deutschland
2.3 Parteck® Mg DC (Magnesiumhydroxidcarbonat), schwer, geeignet für die Verwendung als Excipient EMPROVE® exp Ph Eur, BP, USP, E 504, Artikel Nr. 1.02440, Merck KGaA, Darmstadt, Deutschland
2.4 Fujicalin®, Calciumhydrogenphosphat, wasserfrei, DCPA, USP/NF, EP, JP (Fuji Chemical Industry Co,, Ltd, Japan, bezogen über SEPPIC GmbH, Köln, Deutschland)
2.5 Lactose-Monohydrat (Milchzucker), Spezialsorte für die Tablettierung, geeignet für die Verwendung als Excipient EMPROVE® exp Ph Eur, BP, NF, JP, Artikel Nr. 1.08195, Merck KGaA, Darmstadt, Deutschland
2.6 Starch 1500® (vorverkleisterte Maisstärke) USP/NF, Ph Eur, JPE, IN 516247, Colorcon Limited, UK
2.7 Vivapur® 102 Premium, MCC (Mikrokristalline Cellulose) Ph Eur, NF, JP, JRS Pharma, Rosenberg, Deutschland
2.8 Avicel® PH 102, MCC (Mikrokristalline Cellulose) Ph Eur, NF, JP, FMC BioPolymer, USA
2.9 Emcocel® 90M, MCC (Mikrokristalline Cellulose) Ph Eur, NF, JP, JRS Pharma, Rosenberg, Deutschland

## 3. Pulverförmige Ascorbinsäure (verwendet als Modellwirkstoff)

L(+)-Ascorbinsäure, Ph Eur, USP, NF, Prod, 83568,290, Ch,: 11D180012, VWR, Deutschland

Partikelverteilung bestimmt über Laserbeugung mit Trockendispergierung mit 1 bar Gegendruck:

Angaben µm (Details zum Messverfahren siehe unter Methoden)

| Muster | Dv5 | Dv10 | Dv20 | Dv25 | Dv30 |
|---|---|---|---|---|---|
| Ascorbinsäure | 27,63 | 57,03 | 103,64 | 123,02 | 141,50 |

| Muster | Dv50 | Dv75 | Dv90 | Dv95 |
|---|---|---|---|---|
| Ascorbinsäure | 215,48 | 335,67 | 467,13 | 552,17 |

Partikelverteilung bestimmt über Laserbeugung mit Trockendispergierung mit 2 bar Gegendruck:

Angaben µm (Details zum Messverfahren siehe unter Methoden)

| Muster | Dv5 | Dv10 | Dv20 | Dv25 | Dv30 |
|---|---|---|---|---|---|
| Ascorbinsäure | 24,74 | 52,40 | 100,25 | 120,64 | 140,02 |

| Muster | Dv50 | Dv75 | Dv90 | Dv95 |
|---|---|---|---|---|
| Ascorbinsäure | 217,41 | 346,52 | 505,33 | 634,51 |

Partikelverteilung bestimmt über Laserbeugung mit Trockendisperqierung mit 3 bar Gegendruck:

Angaben μm (Details zum Messverfahren siehe unter Methoden)

| Muster | Dv5 | Dv10 | Dv20 | Dv25 | Dv30 |
|---|---|---|---|---|---|
| Ascorbinsäure | 11,85 | 24,55 | 62,66 | 82,02 | 100,81 |

| Muster | Dv50 | Dv75 | Dv90 | Dv95 |
|---|---|---|---|---|
| Ascorbinsäure | 177,57 | 304,33 | 451,03 | 558,34 |

**Vorgehensweise:**

1. Verpressung der gemahlenen Polyvinylalkohole ohne jegliche Zusätze

2. Herstellung der Abmischungen bestehend aus den verschiedenen direkt verpressbaren Trägerstoffen des Marktes mit der gemahlenen PVA-Type 26-88

3. Verpressung dieser Abmischungen und Tablettencharakterisierung

4. Herstellbeschreibungen der Co-Mischungen aus gemahlenem PVA 26-88 bzw. 40-88 mit der mikrokristallinen Cellulose Vivapur® 102

5. Herstellbeschreibung der Abmischungen der beiden unter 4. erhaltenen Co-Mischungen mit pulverförmiger Ascorbinsäure

6. Verpressung dieser Abmischungen und Tablettencharakterisierung

7. Prüfung der verzögerten in-vitro Freisetzung von Ascorbinsäure aus solchermaßen erhaltenen Presslingen

**A) Versuchsergebnisse:**

**1. Verpressung der gemahlenen PVAs ohne jegliche Zusätze**

Die gemahlenen PVA-Typen 4-88, 18-88, 26-88, 40-88 und 28-99 werden ohne weitere Zusätze (auch kein Schmiermittel) auf einer Tablettiermaschine Korsch EK 0-DMS verpresst. Vor der Verpressung werden zur Beseitigung von eventuellen Zusammenballungen von PVA-Partikeln die gemahlenen PVA-Typen über ein 800 μm-Handsieb (Durchmesser 20 cm; Firma Retsch, Haan, Deutschland) abgelegt.

Als Vergleich dient Parteck® M200 abgemischt mit 1% Parteck® LUB MST. Anmerkung: eine Verpressung des Parteck® M200 ohne jegliches Schmiermittel ist wegen der resultierenden sehr hohen Ausstoßkräfte nicht möglich.

**Tabelle 1:** Tablettierdaten gemahlene PVAs ohne Zusätze

| Legende: | |
|---|---|
| A: Presskraft | [kN] |
| B: Tablettenhärte nach 1 Tag | [N] |
| C: Tablettenmasse | [mg] |

(fortgesetzt)

| Muster | A | | B | C | D | E | F |
|---|---|---|---|---|---|---|---|
| | Soll | Ist | | | | | |
| **PVA 4-88*** | 5 | 5,0 | 17,0 | 470,3 | 5,9 | 59,94 | 237,0 |
| | 10 | 10,1 | 40,8 | 491,8 | 5,6 | 8,94 | 383,5 |
| | 20 | 20,7 | 137,2 | 503,2 | 5,1 | 0,35 | 378,3 |
| | 30 | 30,3 | 194,1 | 504,5 | 5,0 | 0,05 | 322,5 |
| **PVA 18-88*** | 5 | 5,6 | <10 | 409,7 | 5,9 | 100 | 246,4 |
| | 10 | 10,1 | 23,0 | 493,7 | 5,7 | 18,90 | 354,4 |
| | 20 | 19,9 | 89,1 | 499,9 | 5,2 | 1,03 | 382,7 |
| | 30 | 29,9 | 151,1 | 504,0 | 5,0 | 0,14 | 355,7 |
| **PVA 26-88*** | 5 | 7,3 | 23,9 | 444,7 | 5,6 | 23,37 | 318,2 |
| | 10 | 10,7 | 51,1 | 488,8 | 5,4 | 4,98 | 345,7 |
| | 20 | 19,2 | 129,5 | 492,9 | 5,0 | 0,46 | 327,7 |
| | 30 | 30,7 | 191,8 | 490,9 | 4,8 | 0.06 | 275,7 |
| **PVA 40-88*** | 5 | 7,6 | 20,5 | 443,1 | 5,7 | 39,93 | 296,7 |
| | 10 | 10,1 | 33,0 | 490,3 | 5,6 | 9,67 | 321,7 |
| | 20 | 18,8 | 150,8 | 506,6 | 5,0 | 0,65 | 317,7 |
| | 30 | 28,5 | 151,4 | 504,6 | 5,0 | 0,12 | 282,9 |
| **PVA 28-99*** | 5 | 4,7 | <10 | 450,6 | 5,9 | 100 | 169,0 |
| | 10 | 9,7 | 25,5 | 483,9 | 5,5 | 14,22 | 279,5 |
| | 20 | 19,5 | 102,0 | 471,3 | 4,8 | 0,83 | 292,3 |
| | 30 | 30,3 | 178,0 | 472,1 | 4,6 | 0,10 | 263,2 |
| **Parteck® M200** | 5 | 5,2 | 84,1 | 497,8 | 5,1 | 0,21 | 155,8 |
| | 10 | 10,7 | 196,5 | 500,6 | 4,6 | 0,17 | 306,0 |
| | 20 | 20,3 | 340,0 | 499,4 | 4,2 | 0,15 | 513,6 |
| | 30 | 30,0 | 396,7 | 498,3 | 4,0 | 0,16 | 647,6 |

Legende:
D: Tablettenhöhe [mm]
E: Abrieb [%]
F: Ausstoßkraft (N)

* vermahlenes PVA

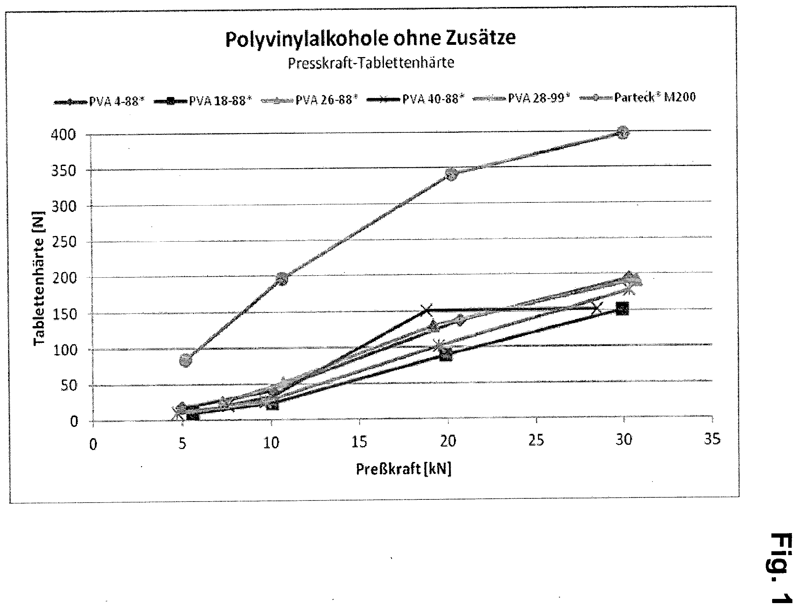

In **Figur 1** sind die Presskraft-Härte-Profile gemäß der Daten aus Tabelle 1 graphisch dargestellt.

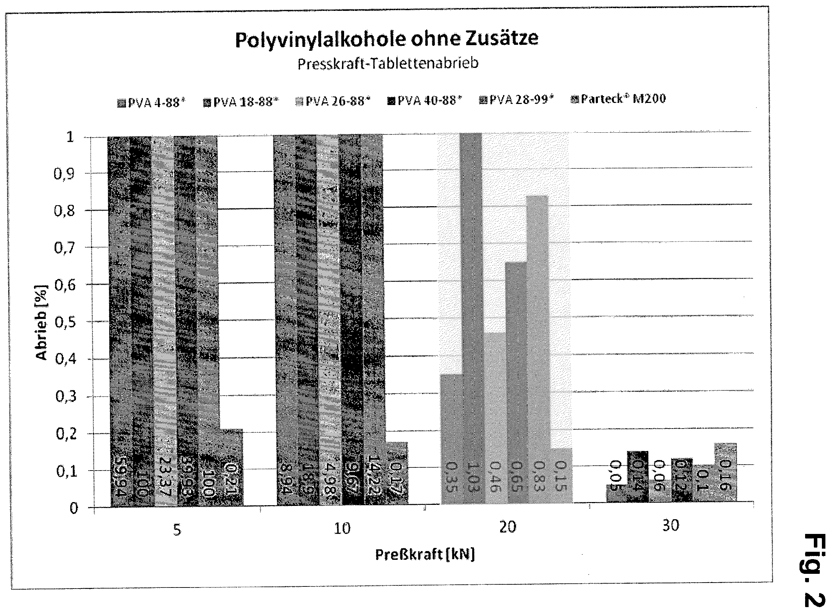

In **Figur 2** sind die Presskraft-Abrieb-Profile gemäß der Daten aus Tabelle 1 graphisch dargestellt.

**Ergebnis:**

a) es ist keine Direktverpressung der gemahlenen PVA-Typen möglich, da Tabletten ungenügender Härten erhalten werden die keine sichere Handhabung ermöglichen (unzureichende Presskraft/Härte-Profile)

b) der Tablettenabrieb, insbesondere bei Einsatz von niedrigen Preßkräften, ist sehr hoch.

c) relativ niedrige Ausstoßkräfte_("Selbstschmierungseffekt") der gemahlenen PVAs; theoretischer Vorteil: stärkere interpartikuläre Bindungskräfte in der Tablette; bei den geprüften PVAs ist dieser Effekt jedoch nicht ausreichend um Tabletten mit ausreichenden Härten und geringem Abrieb zu erhalten.

**2. Herstellung der Abmischungen der direktverpressbaren Trägerstoffe mit der gemahlenen PVA-Type 26-88**

Allgemeine Beschreibung: gemahlenes PVA 26-88 wird durch ein 800 $\mu$m Handsieb abgelegt. 300 g dieser gesiebten Ware werden in ein 2l Turbulamischgefäß eingewogen, 300 g des entsprechenden Trägerstoffes aus A bis I (siehe Tabelle 2) hinzugegeben und 5 min. in einem Turbulamischer T2A gemischt.

**Tabelle 2**:_Zusammensetzung der Beispiele A-C und der Vergleiche D-I

| Zusammensetzung | 50 Gew.% PVA | 50 Gew.% Trägerstoff |
|---|---|---|
| Beispiel A | PVA 26-88* | Vivapur® 102 |
| Beispiel B | PVA 26-88* | Avicel® PH 102 |
| Beispiel C | PVA 26-88* | Emcocel® 90 M |
| Vergleich D | PVA 26-88* | Parteck® SI 150 |
| Vergleich E | PVA 26-88* | Parteck® M 200 |
| Vergleich F | PVA 26-88* | Parteck® Mg DC |
| Vergleich G | PVA 26-88* | Fujicalin® |
| Vergleich H | PVA 26-88* | Lactose |
| Vergleich I | PVA 26-88* | Starch® 1500 |
| * vermahlenes PVA | | |

**Tabelle 3:** Schüttdichte, Stampfdichte und Schüttwinkel der Beispiele A-C

| | Schüttdichte | Stampfdichte | Schüttwinkel |
|---|---|---|---|
| Beispiel A | 0,43 g/ml | 0,58 q/ml | 36,4° |
| Beispiel B | 0,44 g/ml | 0,60 g/ml | 35,3° |
| Beispiel C | 0,45 g/ml | 0,59 g/ml | 35,6° |

**3.Verpressung dieser Abmischungen und Tablettencharakterisierung**

Allgemeine Beschreibung: je 498,75g der oben hergestellten Co-Mischungen aus den Beispielen A-C bzw. den Vergleichen D-I werden in einem Turbulamischgefäß mit 1,25g Magnesiumstearat versetzt, nochmals 5 min. in einem Turbulamischer T2A gemischt und auf einer Exzenterpresse Korsch EK 0-DMS tablettiert.

**Tabelle 4:** Tablettierdaten der Co-Mischungen gemahlenes PVA 26-88 mit Trägerstoffen

| Legende: | | | | | | |
|---|---|---|---|---|---|---|
| A: Presskraft | | [kN] | | | | |
| B: Tablettenhärte nach 1 Tag | | [N] | | | | |
| C: Tablettenmasse | | [mg] | | | | |
| D: Tablettenhöhe | | [mm] | | | | |
| E: Abrieb | | [%] | | | | |
| F: Ausstoßkraft | | (N) | | | | |

| Muster | A | | B | C | D | E | F |
|---|---|---|---|---|---|---|---|
| | Soll | Ist | | | | | |
| **Beispiel A** | 5 | 5,1 | 76,8 | 498,4 | 5,4 | 0,26 | 91,3 |
| | 10 | 10,2 | 171,4 | 502,1 | 4,8 | 0,05 | 91,8 |
| | 20 | 19,5 | 295,7 | 503,4 | 4,5 | 0 | 66,7 |
| | 30 | 30,0 | 354,5 | 502,5 | 4,4 | 0 | 58,6 |
| **Beispiel B** | 5 | 4,9 | 70,2 | 501,8 | 5,4 | 0,49 | 85,9 |

(fortgesetzt)

| Muster | A | | B | C | D | E | F |
|---|---|---|---|---|---|---|---|
| | Soll | Ist | | | | | |
| | 10 | 9,6 | 153,1 | 506,1 | 4,9 | 0,16 | 87,3 |
| | 20 | 18,4 | 267,3 | 506,6 | 4,5 | 0,07 | 61,1 |
| | 30 | 28,6 | 325,1 | 506,8 | 4,4 | 0,04 | 52,1 |
| Beispiel C | 5 | 4,9 | 71,0 | 494,2 | 5,5 | 0,39 | 90,9 |
| | 10 | 10,2 | 159,6 | 497,0 | 4,9 | 0,06 | 92,3 |
| | 20 | 20,0 | 273,6 | 496,8 | 4,5 | 0 | 64,8 |
| | 30 | 30,4 | 318,0 | 498,2 | 4,4 | 0 | 57,3 |
| Vergleich D | 5 | 5,0 | 31,0 | 498,2 | 5,4 | 3,86 | 66,6 |
| | 10 | 9,9 | 86,0 | 502,8 | 4,9 | 0,37 | 94,1 |
| | 20 | 20,5 | 170,0 | 503,6 | 4,5 | 0,08 | 78,6 |
| | 30 | 30,8 | 188,5 | 503,4 | 4,5 | 0,06 | 64,8 |
| Vergleich E | 5 | 5,0 | 17,1 | 493,0 | 5,6 | 17,05 | 84,2 |
| | 10 | 10,0 | 51,5 | 499,8 | 5,1 | 1,12 | 138,8 |
| | 20 | 20,3 | 137,6 | 501,3 | 4,7 | 0,21 | 162,9 |
| | 30 | 29,7 | 178,0 | 500,1 | 4,6 | 0,16 | 150,9 |
| Vergleich F | 5 | 6,1 | 22,1 | 482,7 | 5,6 | 7,30 | 107,7 |
| | 10 | 10,2 | 50,1 | 501,4 | 5,2 | 1,28 | 133,2 |
| | 20 | 20,9 | 137,4 | 505,0 | 4,7 | 0,13 | 149,0 |
| | 30 | 31,2 | 224,3 | 501,6 | 4,5 | 0,01 | 144,0 |
| Vergleich G | 5 | 5,0 | 22,7 | 492,7 | 4,9 | 9,55 | 121,0 |
| | 10 | 10,3 | 48,6 | 495,0 | 4,5 | 1,42 | 148,5 |
| | 20 | 20,6 | 115,2 | 494,5 | 4,1 | 0,27 | 126,2 |
| | 30 | 29,6 | 161,6 | 492,1 | 3,9 | 0,10 | 102,0 |
| Vergleich H | 5 | 4,9 | <10 | 374,7 | 5,1 | n. b. | 57,3 |
| | 10 | 10,2 | 16,7 | 488,2 | 5,0 | 100 | 98,4 |
| | 20 | 19,9 | 50,2 | 495,3 | 4,6 | 2,00 | 127,3 |
| | 30 | 29,0 | 77,4 | 497,3 | 4,5 | 0,50 | 135,1 |
| Vergleich I | 5 | 5,0 | < 10 | 468,2 | 5,5 | 100 | 54,8 |
| | 10 | 9,8 | 28,9 | 492,3 | 5,1 | 10,49 | 69,1 |
| | 20 | 19,6 | 77,3 | 494,1 | 4,7 | 0,78 | 57,2 |
| | 30 | 30,0 | 98,7 | 494,3 | 4,6 | 0,30 | 50,6 |
| Parteck® M200 | 5 | 5,2 | 84,1 | 497,8 | 5,1 | 0,21 | 155,8 |
| | 10 | 10,7 | 196,5 | 500,6 | 4,6 | 0,17 | 306,0 |
| | 20 | 20,3 | 340,0 | 499,4 | 4,2 | 0,15 | 513,6 |
| | 30 | 30,0 | 396,7 | 498,3 | 4,0 | 0,16 | 647,6 |

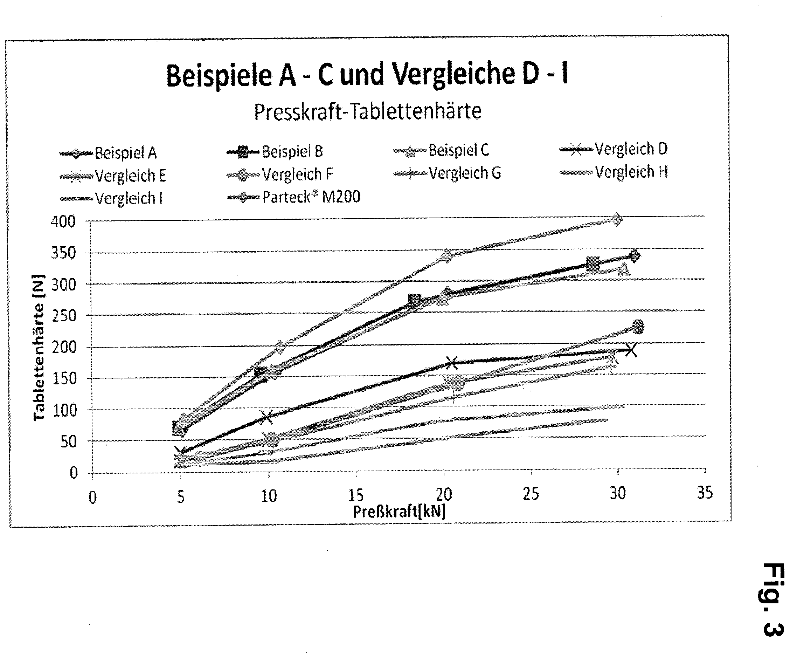

**In Figur 3 sind die** Presskraft-Härte-Profile der Daten gemäß der Zusammensetzungen aus Tabelle 4 dargestellt.

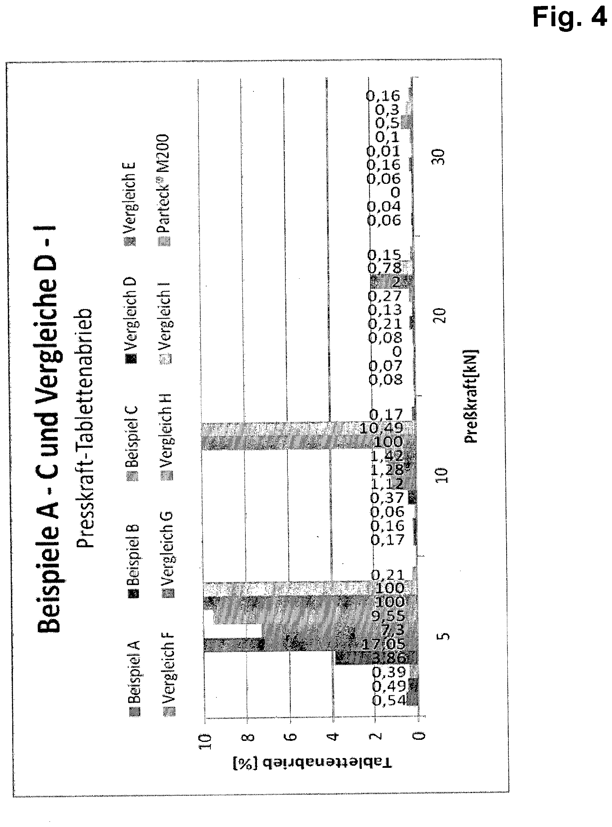

**In Figur 4 sind die** Presskraft-Abrieb-Profile graphisch anhand der Daten aus Tabelle 4 dargestellt.

**Ergebnis:**

a) nur die Co-Mischungen auf Basis von gemahlenem PVA 26-88 mit den drei geprüften MCC-Typen (Beispiele A-C) liefern bei allen 4 geprüften Presskräften Tabletten mit ausreichenden Härten und kommen in ihren Kompressibilitäten dem internen Standard Parteck® M 200 sehr nahe; alle anderen Co-Mischungen zeigen bei den gleichen Presskräften deutlich geringere Tablettenhärten

b) Tabletten auf Basis der Beispiele A-C zeigen insbesondere bei niedrigen Presskräften eine gegenüber den anderen Matrices reduzierte Friabilität.

## 4. Herstellungbeschreibung der Co-Mischungen aus gemahlenem PVA 26-88 und gemahlenem PVA 40-88 mit Vivapur® 102

Beispiel A: gemahlenes PVA 26-88 wird durch ein 800 $\mu$m Handsieb abgelegt. 300 g der gesiebten Ware werden in ein 2l Turbulamischgefäß eingewogen, mit 300 g Vivapur® Type 102 versetzt und 5 min. in einem Turbulamischer T2A gemischt.

Beispiel D:gemahlenes PVA 40-88 wird durch ein 800 $\mu$m Handsieb abgelegt. 300 g der gesiebten Ware wird in ein 2l Turbulamischgefäß eingewogen, mit 300 g Vivapur® Type 102 versetzt und 5 min. in einem Turbulamischer T2A gemischt.

**Tabelle 5:** Zusammensetzung der Beispiele A und D

| Zusammensetzung | 50 Gew.% PVA | 50 Gew.% Trägerstoff |
|---|---|---|
| Beispiel A | PVA 26-88* | Vivapur® 102 |
| Beispiel D | PVA 40-88* | Vivapur® 102 |
| * vermahlenes PVA | | |

**Tabelle 6:**_Schüttdichte, Stampfdichte und Schüttwinkel der Beispiele A und D

| | Schüttdichte | Stampfdichte | Schüttwinkel |
|---|---|---|---|
| Beispiel A | 0,43 g/ml | 0,58 g/ml | 36,4° |
| Beispiel D | 0,43 g/ml | 0,59 g/ml | 36,3° |

**Tabelle 7: Tablettierdaten Beispiel A und Beispiel D**

Legende:
A: Presskraft [kN]
B: Tablettenhärte nach 1 Tag [N]
C: Tablettenmasse [mg]
D: Tablettenhöhe [mm]
E: Abrieb [%]
F: Ausstoßkraft (N)

| Muster | A | | B | C | D | E | F |
|---|---|---|---|---|---|---|---|
| | Soll | Ist | | | | | |
| **Beispiel A** | 5 | 5,1 | 76,8 | 498,4 | 5,4 | 0,26 | 91,3 |
| | 10 | 10,2 | 171,4 | 502,1 | 4,8 | 0,05 | 91,8 |
| | 20 | 19,5 | 295,7 | 503,4 | 4,5 | 0 | 66,7 |
| | 30 | 30,0 | 354,5 | 502,5 | 4,4 | 0 | 58,6 |
| **Beispiel D** | 5 | 5,0 | 64,2 | 500,4 | 5,4 | 0,49 | 76 |
| | 10 | 10,3 | 146,9 | 505,7 | 4,9 | 0,15 | 90 |
| | 20 | 20,1 | 247,4 | 506,0 | 4,5 | 0,08 | 62 |
| | 30 | 32,0 | 296,6 | 506,0 | 4,5 | 0,07 | 91 |

## 5. Herstellbeschreibung der Abmischungen der beiden unter 4. erhaltenen Co-Mischungen mit pulverförmiger Ascorbinsäure

Muster 1: 450 g Co-Mischung Beispiel A werden mit 150 g Ascorbinsäure versetzt und 5 min. im Turbulamischer T2A gemischt. Auf 498,75 g dieser Mischung wird über ein 250 μm Sieb 1,25 g Magnesiumstearat aufgesiebt und 5 Minuten im Turbulamischer T2A gemischt

Muster 2: 450 g Co-Mischung Beispiel D werden mit 150 g Ascorbinsäure versetzt und 5 min. im Turbulamischer T2A gemischt. Auf 498,75 g dieser Mischung wird über ein 250 μm Sieb 1,25 g Magnesiumstearat aufgesiebt und 5 Minuten im Turbulamischer T2A gemischt

**6.Verpressung der Muster 1 und 2 sowie Tablettencharakterisierung**

**Tabelle 8: Tablettierdaten der Muster 1 und 2**

| Legende: | |
|---|---|
| A: Presskraft | [kN] |
| B: Tablettenhärte nach 1 Tag | [N] |
| C: Tablettenmasse | [mg] |
| D: Tablettenhöhe | [mm] |
| E: Abrieb | [%] |
| F: Ausstoßkraft | (N) |

| Muster | A | | B | C | D | E | F |
|---|---|---|---|---|---|---|---|
| | Soll | Ist | | | | | |
| **Muster 1** | 5 | 5,3 | 34,8 | 501,6 | 5,1 | 3,19 | 73,4 |
| | 10 | 10,0 | 74,4 | 504,6 | 4,7 | 0,61 | 88,2 |
| | 20 | 20,0 | 140,0 | 504,5 | 4,3 | 0,21 | 88,0 |
| | 30 | 30,5 | 173,7 | 505,2 | 4,2 | 0,14 | 87,6 |
| **Muster 2** | 5 | 5,1 | 25,5 | 498,0 | 5,1 | 7,15 | 73,6 |
| | 10 | 11,2 | 61,9 | 501,1 | 4,6 | 0,75 | 95,6 |
| | 20 | 20,8 | 125,8 | 503,5 | 4,4 | 0,12 | 96,0 |
| | 30 | 31,1 | 157,6 | 506,3 | 4,2 | 0,08 | 95,7 |

**Ergebnis:**

1. Auch in Kombination mit einer als schlecht direkt verpressbar geltenden pulverförmigen Ascorbinsäure werden unter Verwendung der erfindungsgemäßen Co-Mischungen Beispiel A und D Tabletten ausreichender Härte und geringer Friabilität erhalten welche sich problemlos handhaben lassen; dadurch kann auf die Verwendung von sonst üblichen direkt verpressbaren Ascorbinsäuretypen verzichtet werden

2. Die Ausstoßkräfte der Mischungen mit Beispiel A und Beispiel D sind - auch bei der nur sehr geringen Menge an zugesetztem Magnesiumstearat- ungewöhnlich niedrig; dies bedingt einen geringeren Verschleiß der Stempelwerkzeuge und Tablettiermaschinen.

3. Durch die verhältnismäßig geringe Menge an zugesetztem Magnesiumstearat wird die angestrebte retardierte Wirkstofffreisetzung im Wesentlich nur durch die Mengen und Eigenschaften des verwendeten PVAs bestimmt; der bekanntlich störende Einfluß des hydrophoben Magnesiumstearats auf das Wirkstofffreisetzungsverhaltens wird minimiert.

**7. Prüfung der verzögerten in-vitro Freisetzung von Ascorbinsäure aus solchermaßen erhaltenen Presslingen**

**Tabelle 9:** Ergebnisse der Ascorbinsäurefreisetzung aus Retardtabletten des Musters 1 und Muster 2 (gepresst bei 20 kN Preßkraft)

| (Angaben in Gew.% der freigesetzten Ascorbinsäuremenge bezogen auf eine zu erwartende Gesamtmenge an Ascorbinsäure/Tablette, Vermessung von je 6 Tabletten pro Muster) | | | | | | |
|---|---|---|---|---|---|---|
| | Muster 1 (Tabletten gepresst mit 20 kN Presskraft) | | | Muster 2 (Tabletten gepresst mit 20 kN Presskraft) | | |
| Zeit (min.) | Min | Max | Mittelwert | Min | Max | Mittelwert |
| 0 | 0 | 0 | 0 | 0 | 0 | 0 |
| 15 | 14 | 20 | 17 | 15 | 18 | 16 |
| 30 | 21 | 28 | 24 | 22 | 25 | 24 |
| 45 | 26 | 34 | 30 | 27 | 32 | 30 |
| 60 | 31 | 40 | 35 | 32 | 37 | 35 |
| 120 | 46 | 57 | 51 | 46 | 53 | 50 |
| 180 | 60 | 72 | 65 | 58 | 66 | 63 |
| 240 | 71 | 84 | 77 | 67 | 77 | 73 |
| 300 | 81 | 91 | 86 | 72 | 82 | 78 |
| 360 | 89 | 98 | 93 | 78 | 89 | 85 |
| 420 | 94 | 101 | 97 | 86 | 93 | 90 |
| 480 | 97 | 103 | 100 | 90 | 96 | 94 |
| 540 | 98 | 104 | 101 | 96 | 102 | 99 |
| 600 | 98 | 104 | 101 | 98 | 103 | 101 |
| 660 | 98 | 104 | 101 | 99 | 103 | 101 |
| 720 | 98 | 104 | 101 | 100 | 103 | 102 |

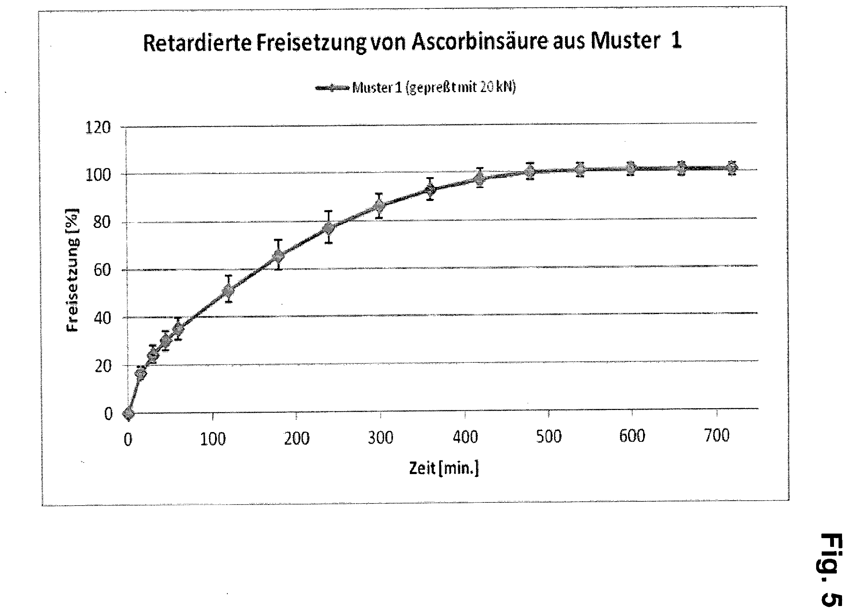

In **Figur 5** ist die Freisetzung von Ascorbinsäure aus Retardtabletten gemäß Muster 1, charakterisiert durch Daten aus Tabelle 9, graphisch dargestellt.

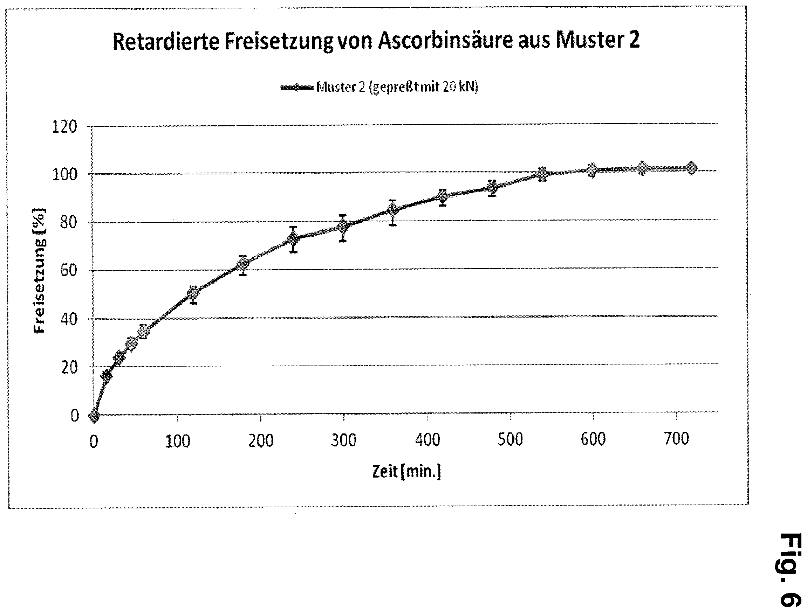

In **Figur 6** ist die Freisetzung von Ascorbinsäure aus Retardtabletten gemäß Muster 2 graphisch dargestellt anhand der Daten aus Tabelle 9.

**Ergebnis:** eine retardierte in-vitro Freisetzung des Modellwirkstoffes Ascorbinsäure ist über mehrere Stunden möglich

**B) Schlussfolgerung**

1. Die Co-Mischungen aus gemahlenem PVA mit MCC führen zu sehr gut direkt tablettierbaren Tablettenmatrices. Schon bei verhältnismäßig niedrigen Presskräften lassen sich Tabletten mit ausreichender Härte und mechanischer Stabilität herstellen.

2. Mit diesen Matrices lassen sich auch per se als schlecht tablettierbar geltende Wirkstoffe in einem Direkttablettierungsprozess zu Tabletten mit guten galenischen Eigenschaften, insbesondere hinsichtlich Härte und mechanischer Stabilität, verarbeiten.

3. Mit Hilfe dieser Matrices können durch Direkttablettierung Retardtabletten mit einer über mehrere Stunden andauernden Wirkstofffreisetzung auf schnelle und problemlose Weise herstellt werden.

**Patentansprüche**

1. Direkt verpressbare Zusammensetzung mit verlängerter Wirkstofffreisetzung, enthaltend eine Co-Mischung aus mikrokristallinen Cellulosen (MCCs) und Polyvinylalkoholen (PVAs).

**2.** Direkt verpressbare Zusammensetzung gemäß Anspruch 1, enthaltend eine Co-Mischung aus mikrokristallinen Cellulosen (MCCs) und Polyvinylalkoholen (PVAs), wobei letztere die Anforderungen der Pharmakopöen PhEur, USP oder JPE erfüllen.

**3.** Direkt verpressbare Zusammensetzung gemäß Anspruch 1 oder 2, enthaltend Polyvinylalkohole (PVAs) der Typen 18-88, 26-88 und 40-88 und alle dazwischen liegenden Qualitäten gemäß den Anforderungen der Pharmakopöen PhEur, USP oder JPE, einschließlich des Typs 28-99 gemäß den Anforderungen der JPE oder PhEur.

**4.** Direkt verpressbare Zusammensetzung gemäß einem oder mehreren der Ansprüche 1 bis 3, enthaltend Polyvinylalkohole (PVAs), welche der PhEur entsprechen und welche durch Polymerisation von Vinylacetat und durch anschließende teilweise oder nahezu vollständige Hydrolyse des Polyvinylacetats erhalten worden sind.

**5.** Direkt verpressbare Zusammensetzung gemäß Anspruch 1, 2, 3 oder 4, enthaltend Polyvinylalkohole (PVAs), welche durch 85% - 89%ige Hydrolyse erhalten worden sind.

**6.** Direkt verpressbare Zusammensetzung gemäß einem oder mehreren der Ansprüche 1 bis 5, enthaltend Polyvinylalkohole (PVAs) mit einer durchschnittlichen relativen Molekülmasse im Bereich zwischen 20 000 und 150 000 g/mol, die nach PhEur eine Viskosität im Bereich von 3 - 70 mPa.s, (gemessen in einer 4%igen Lösung bei 20 °C) aufweisen.

**7.** Direkt verpressbare Zusammensetzung gemäß einem oder mehreren der Ansprüche 1 bis 6, enthaltend Polyvinylalkohole (PVAs), welche eine Esterzahl von nicht größer als 280 mg KOH/g (Hydrolysegrad >72.2 mol%) aufweisen.

**8.** Direkt verpressbare Zusammensetzung gemäß einem oder mehreren der Ansprüche 1 bis 7, welche Polyvinylalkohole (PVAs) als wasserlösliches Harz enthält, das nach USP durch die Formel

$$(C_2H_4O)_n$$

charakterisiert ist, worin

n eine ganze Zahl im Bereich von 500 und bis 5 000 bedeutet.

**9.** Direkt verpressbare Zusammensetzung gemäß einem oder mehreren der Ansprüche 1 bis 8, enthaltend PVA und MCC in einer Co-Mischung im Verhältnis in einem Bereich 2 : 1 bis 1:2, bevorzugt in einem Verhältnis in einem Bereich von 2 : 1 bis 1:1.

**10.** Direkt verpressbare Zusammensetzung gemäß einem oder mehreren der Ansprüche 1 bis 9, **dadurch gekennzeichnet, dass** die Co-Mischung aus PVA mit MCCs Schüttdichten im Bereich von 0,40 - 0,48 g/ml bei Stampfdichten im Bereich von 0,55-0,63 g/ml aufweisen.

**11.** Tablette, enthaltend eine Zusammensetzung gemäß einem oder mehreren der Ansprüche 1 bis 10, welche bereits bei Einsatz einer Presskraft von 19,5 kN zu Tabletten mit einer Tablettenhärte von 295,7 N führen und eine Ausstoßkraft von etwa 66,7 N erfordern.

**12.** Wirkstoffhaltige Tablette mit verlängerter Wirkstofffreisetzung von mehreren Stunden, enthaltend eine Co-Mischung aus Polyvinylalkoholen (PVAs) und mikrokristallinen Cellulosen (MCCs) gemäß einem oder mehreren der Ansprüche 1 bis 10.

**13.** Wirkstoffhaltige Tablette gemäß Anspruch 12, enthaltend eine direktverpressbare Zusammensetzung in Form einer Co-Mischung gemäß einem oder mehreren der Ansprüche 1 bis 10 in einer Menge von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette.

**14.** Wirkstoffhaltige Tablette gemäß Anspruch 12 oder 13, welche bereits bei Einsatz von niedrigen Preßkräften besonders hohe Tablettenhärten aufweisen und geringe Ausstoßkräfte erfordern.

**15.** Wirkstoffhaltige Tablette gemäß einem der Anspruch 12 bis 14, welche geringe Friabilitäten von weniger als 1 Gew.-% zeigen, bevorzugt von weniger als 0,5 Gew.-%, insbesondere von weniger als 0,1 Gew.-%.

**16.** Wirkstoffhaltige Tablette gemäß einem oder mehreren der Ansprüche 11 bis 15 mit verzögerter Wirkstofffreisetzung von mindestens 2 Stunden, bevorzugt über mindestens 6 Stunden, besonders bevorzugter von mindestens 8 Stunden, insbesondere bevorzugt von mindestens 10 Stunden, und ganz besonders bevorzugt von mindestens 12 Stunden.

**17.** Wirkstoffhaltige Tablette gemäß einem oder mehreren der Ansprüche 11 bis 16 mit verzögerter Wirkstofffreisetzung, enthaltend Wirkstoffe der BCS Klasse I entweder allein oder in Kombination mit anderen Wirkstoffen.

**18.** Verfahren zur Herstellung von direkt verpressbaren Zusammensetzungen gemäß einem oder mehreren der Ansprüche 1 bis 11 mit verlängerter Wirkstofffreisetzung, enthaltend eine Co-Mischung aus mikrokristallinen Cellulosen (MCCs) und Polyvinylalkoholen (PVAs), **dadurch gekennzeichnet, dass** Polyvinylalkohol zu einem feinkörnigen Pulver gemahlen und durch ein $800 \mu m$ Sieb gesiebt wird, und mit microkristalliner Cellulose (MCCs) mit mittlerer Partikelgröße $D_{v50}$ im Bereich von 60 bis 250 $\mu m$, und einer Schüttdichte im Bereich von 0,22 bis 0,38 g/cm$^3$ intensiv vermischt wird.

**Claims**

**1.** Directly compressible composition having extended release of active compound, comprising a co-mixture of microcrystalline celluloses (MCCs) and polyvinyl alcohols (PVAs).

**2.** Directly compressible composition according to Claim 1, comprising a co-mixture of microcrystalline celluloses (MCCs) and polyvinyl alcohols (PVAs), where the latter meet the requirements of the pharmacopoeias PhEur, USP or JPE.

**3.** Directly compressible composition according to Claim 1 or 2, comprising polyvinyl alcohols (PVAs) of grades 18-88, 26-88 and 40-88 and all grades in between in accordance with the requirements of the pharmacopoeias PhEur, USP or JPE, including grade 28-99 in accordance with the requirements of the JPE or PhEur.

**4.** Directly compressible composition according to one or more of Claims 1 to 3, comprising polyvinyl alcohols (PVAs) which conform to PhEur and which have been obtained by polymerisation of vinyl acetate and by subsequent partial or virtually complete hydrolysis of the polyvinyl acetate.

**5.** Directly compressible composition according to Claim 1, 2, 3 or 4, comprising polyvinyl alcohols (PVAs) which have been obtained by 85% - 89% hydrolysis.

**6.** Directly compressible composition according to one or more of Claims 1 to 5, comprising polyvinyl alcohols (PVAs) having an average relative molecular weight in the range between 20,000 and 150,000 g/mol which have a viscosity in accordance with PhEur in the range 3 - 70 mPa.s (measured in a 4% solution at 20°C).

**7.** Directly compressible composition according to one or more of Claims 1 to 6, comprising polyvinyl alcohols (PVAs) which have an ester value of not greater than 280 mg of KOH/g (degree of hydrolysis > 72.2 mol%).

**8.** Directly compressible composition according to one or more of Claims 1 to 7, which comprises polyvinyl alcohols (PVAs) as water-soluble resin, which is **characterised in** accordance with USP by the formula

$$(C_2H_4O)_n,$$

in which

n denotes an integer in the range from 500 to 5,000.

**9.** Directly compressible composition according to one or more of Claims 1 to 8, comprising PVA and MCC in a co-mixture in a ratio in the range 2 : 1 to 1:2, preferably in a ratio in the range 2 : 1 to 1:1.

**10.** Directly compressible composition according to one or more of Claims 1 to 9, **characterised in that** the co-mixture of PVA with MCCs has a bulk density in the range 0.40 - 0.48 g/ml with tapped densities in the range 0.55-0.63 g/ml.

**11.** Tablet comprising a composition according to one or more of Claims 1 to 10 which, even on use of a pressing force of 19.5 kN, results in tablets having a tablet hardness of 295.7 N and requires an ejection force of about 66.7 N.

12. Active-compound-containing tablet having extended release of active compound over several hours, comprising a co-mixture of polyvinyl alcohols (PVAs) and microcrystalline celluloses (MCCs) in accordance with one or more of Claims 1 to 10.

13. Active-compound-containing tablet according to Claim 12, comprising a directly compressible composition in the form of a co-mixture in accordance with one or more of Claims 1 to 10 in an amount of 1 - 99% by weight, preferably in an amount of 5 - 95% by weight, very particularly preferably in an amount of 10 - 90% by weight, based on the total weight of the tablet.

14. Active-compound-containing tablet according to Claim 12 or 13, which has a particularly high tablet hardness, even on use of low pressing forces, and requires low ejection forces.

15. Active-compound-containing tablet according to one of Claim 12 to 14, which exhibits a low friability of less than 1% by weight, preferably of less than 0.5% by weight, in particular of less than 0.1% by weight.

16. Active-compound-containing tablet according to one or more of Claims 11 to 15 having delayed release of active compound of at least 2 hours, preferably over at least 6 hours, particularly preferably of at least 8 hours, especially preferably of at least 10 hours, and very particularly preferably of at least 12 hours.

17. Active-compound-containing tablet according to one or more of Claims 11 to 16 having delayed release of active compound, comprising active compounds in BCS class I, either alone or in combination with other active compounds.

18. Process for the preparation of directly compressible compositions according to one or more of Claims 1 to 11 having extended release of active compound, comprising a co-mixture of microcrystalline celluloses (MCCs) and polyvinyl alcohols (PVAs), **characterised in that** polyvinyl alcohol is ground to give a fine-grained powder and sieved through an 800 $\mu$m sieve, and mixed intensively with microcrystalline cellulose (MCCs) having an average particle size $D_{v50}$ in the range from 60 to 250 $\mu$m, and a bulk density in the range from 0.22 to 0.38 g/cm$^3$.

**Revendications**

1. Composition directement compressible ayant une libération prolongée de composé actif, comprenant un co-mélange de celluloses microcristallines (MCC) et alcools polyvinyliques (PVA).

2. Composition directement compressible selon la revendication 1, comprenant un co-mélange de celluloses microcristallines (MCC) et d'alcools polyvinyliques (PVA), où ces derniers remplissent les exigences des pharmacopées PhEur, USP ou JPE.

3. Composition directement compressible selon la revendication 1 ou 2, comprenant des alcools polyvinyliques (PVA) de qualités 18-88, 26-88 et 40-88 et toutes les qualités intermédiaires conformément aux exigences des pharmacopées PhEur, USP ou JPE, y compris la qualité 28-99 conformément aux exigences de JPE ou PhEur.

4. Composition directement compressible selon l'une ou plusieurs parmi les revendications 1 à 3, comprenant des alcools polyvinyliques (PVA) qui sont conformes à PhEur et qui ont été obtenus par polymérisation d'acétate de vinyle et par hydrolyse ultérieure partielle ou pratiquement complète de l'acétate de polyvinyle.

5. Composition directement compressible selon la revendication 1, 2, 3 ou 4, comprenant des alcools polyvinyliques (PVA) qui ont été obtenus par 85% - 89% d'hydrolyse.

6. Composition directement compressible selon l'une ou plusieurs parmi les revendications 1 à 5, comprenant des alcools polyvinyliques (PVA) ayant un poids moléculaire relatif moyen dans la plage comprise entre 20 000 et 150 000 g/mol, qui possèdent une viscosité conformément à PhEur dans la plage de 3 - 70 mPa.s (mesurée dans une solution à 4% et à 20°C).

7. Composition directement compressible selon l'une ou plusieurs parmi les revendications 1 à 6, comprenant des alcools polyvinyliques (PVA) qui possèdent un indice d'ester non supérieur à 280 mg de KOH/g (degré d'hydrolyse > 72,2 mol%).

**8.** Composition directement compressible selon l'une ou plusieurs parmi les revendications 1 à 7, comprenant des alcools polyvinyliques (PVA) sous forme de résine hydrosoluble, qui est **caractérisée** conformément à l'USP par la formule

$$(C_2H_4O)_n,$$

où

n désigne un nombre entier dans la plage allant de 500 à 5000.

**9.** Composition directement compressible selon l'une ou plusieurs parmi les revendications 1 à 8, comprenant du PVA et de la MCC dans un co-mélange selon un rapport dans la plage allant de 2:1 à 1:2, préférablement selon un rapport dans la plage allant de 2:1 à 1:1.

**10.** Composition directement compressible selon l'une ou plusieurs parmi les revendications 1 à 9, **caractérisée en ce que** le co-mélange de PVA avec des MCC possède une densité apparente dans la plage de 0,40-0,48 g/ml avec des densités après tassement dans la plage de 0,55-0,63 g/ml.

**11.** Comprimé comprenant une composition selon l'une ou plusieurs parmi les revendications 1 à 10 qui, même lors de l'utilisation d'une force de pression de 19,5 kN, conduit à des comprimés ayant une dureté de comprimé de 295,7 N et nécessite une force d'éjection d'environ 66,7 N.

**12.** Comprimé contenant un composé actif ayant une libération prolongée de composé actif sur plusieurs heures, comprenant un co-mélange d'alcools polyvinyliques (PVA) et de celluloses microcristallines (MCC) selon l'une ou plusieurs parmi les revendications 1 à 10.

**13.** Comprimé contenant un composé actif selon la revendication 12, comprenant une composition directement compressible sous la forme d'un co-mélange selon l'une ou plusieurs parmi les revendications 1 à 10 selon une quantité de 1 - 99% en poids, préférablement selon une quantité de 5 - 95% en poids, très particulièrement préférablement selon une quantité de 10 - 90% en poids, sur la base du poids total du comprimé.

**14.** Comprimé contenant un composé actif selon la revendication 12 ou 13, qui possède une dureté de comprimé particulièrement élevée, même lors de l'utilisation de forces de pression faibles, et nécessite des forces d'éjection faibles.

**15.** Comprimé contenant un composé actif selon l'une des revendications 12 à 14, qui présente une friabilité faible inférieure à 1% en poids, préférablement inférieure à 0,5% en poids, en particulier inférieure à 0,1% en poids.

**16.** Comprimé contenant un composé actif selon l'une ou plusieurs parmi les revendications 11 à 15, ayant une libération retardée de composé actif d'au moins 2 heures, préférablement sur au moins 6 heures, particulièrement préférablement d'au moins 8 heures, particulièrement préférablement d'au moins 10 heures, et très particulièrement préférablement d'au moins 12 heures.

**17.** Comprimé contenant un composé actif selon l'une ou plusieurs parmi les revendications 11 à 16, ayant une libération retardée de composé actif, comprenant des composés actifs de la classe I du BCS, seuls ou bien en combinaison avec d'autres composés actifs.

**18.** Procédé de préparation de compositions directement compressibles selon l'une ou plusieurs parmi les revendications 1 à 11, ayant une libération prolongée de composé actif, comprenant un co-mélange de celluloses microcristallines (MCC) et d'alcools polyvinyliques (PVA), **caractérisé en ce qu'**un alcool polyvinylique est broyé pour donner une poudre à grains fins et tamisé sur un tamis de 800 $\mu$m, puis mélangé de manière intensive avec une cellulose microcristalline (MCC) ayant une taille de particule moyenne $D_{v50}$ dans la plage allant de 60 à 250 $\mu$m, et une densité apparente dans la plage allant de 0,22 à 0,38 g/cm$^3$.

## Fig. 1

Polyvinylalkohole ohne Zusätze
Presskraft-Tablettenhärte

Fig. 2

EP 3 174 530 B1

# Beispiele A - C und Vergleiche D - I

## Presskraft-Tablettenhärte

Fig. 3

EP 3 174 530 B1

# Beispiele A - C und Vergleiche D - I

## Presskraft-Tablettenabrieb

▦ Beispiel A     ▦ Beispiel B     ▨ Beispiel C     ▦ Vergleich D     ▨ Vergleich E

▨ Vergleich F     ▨ Vergleich G     ▨ Vergleich H     ▨ Vergleich I     ▨ Parteck® M200

Fig. 4

Retardierte Freisetzung von Ascorbinsäure aus Muster 1

—◇— Muster 1 (gepreßt mit 20 kN)

Fig. 5

**Fig. 6**

Retardierte Freisetzung von Ascorbinsäure aus Muster 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **AMIDON GL ; LENNERNAS H ; SHAH VP ; CRISON JR.** A theoretical basis for a biopharmaceutic drug classification: the correlation of in vitro drug product dissolution and in vivo bioavailability. *Pharm Res.,* 1995, vol. 12, 413

- **S. BRUNAUER et al.** BET Surface Area by Nitrogen Absorption. *Journal of American Chemical Society,* 1983, vol. 60, 9
- Friabilität von nicht überzogenen Tabletten. Geräteparameter und Ausführung der Messungen gemäß Ph.Eur